# EUROPEAN PATENT APPLICATION

(11) **EP 4 643 856 A1**
(43) Date of publication of application: **05.11.2025**
(21) Application number: 24305705.6
(22) Date of filing: 03.05.2024
(51) Int. Cl.: A61K 31/403, A61P 3/04, A61P 3/10, A61P 21/00, A61P 25/28, A61P 43/00

(54) **CARBAZOLE DERIVATIVES USED AS NEUROPROTECTANTS AND IN THE TREATMENT OF DISORDERS WITH REDUCED NAD METABOLISM**

(71) Applicant: Centre National de la Recherche Scientifique, 75016 Paris (FR); Université de Caen Normandie, 14000 Caen (FR); Université Grenoble Alpes, 38400 Saint-Martin-d'Hères (FR); Institut National de la Santé et de la Recherche Médicale, 75013 Paris (FR)
(72) Inventor: LAFANECHERE, Laurence, 38000 GRENOBLE (FR); BOSC, Lauriane, 38000 GRENOBLE (FR); DALLEMAGNE, Patrick, 14260 SEULLINE (FR); SUZANNE, Peggy, CAIRON 14610 (FR)
(74) Representative: Novagraaf Technologies

(57) **Abstract**

The invention concerns a compound of formula (I) 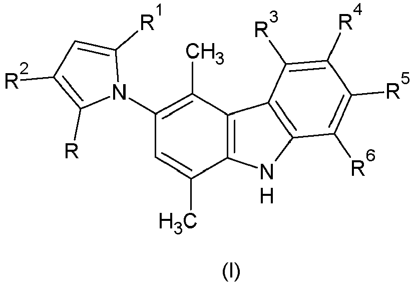 wherein R, R¹ and R² are independently selected form the group consisting of:
- a hydrogen atom,
- a halogen atom,
- an alkyl group, linear, cyclic or branched, saturated or unsaturated, possibly substituted, comprising from 1 to 10 carbon atoms,
- an acyl group comprising from 1 to 10 carbon atoms,
- a carboxyl group,
- an amido group comprising from 1 to 10 carbon atoms, and
- an imino group, possibly substituted by an alkyl group, linear, cyclic or branched, saturated or unsaturated, and

wherein R³, R⁴, R⁵ and R⁶ are independently selected form the group consisting of:
- a hydrogen atom,
- a halogen atom,
- a hydroxyl group,
- an alkyl group, linear, cyclic or branched, saturated or unsaturated, possibly substituted, comprising from 1 to 10 carbon atoms,
- an alkoxy group comprising from 1 to 10 carbon atoms,
- an acyl group comprising from 1 to 10 carbon atoms,
- a carbonate group from 1 to 10 carbon atoms,
- a carboxyl group, and
- a cyano group,
for use as neuroprotectants, or in the treatment of disorders with reduced NAD metabolism.

## Description

The present invention belongs to the fields of human and animal health and notably cancerology and neurology.

The present invention concerns carbazole derivatives of formula (I) for use as neuroprotectants, or in the treatment of disorders with reduced NAD metabolism.

The present invention also concerns pharmaceutical compositions comprising such compounds of formula (I) for use as neuroprotectants, or in the treatment of disorders with reduced NAD metabolism.

### TECHNOLOGICAL BACKGROUND

Patent application WO2021069736 discloses a family of compounds providing improved treatments against cancers and in particular, providing a solution to the technical problem of reducing the toxicity of compounds stabilizing cell microtubules, and in particular of paclitaxel. This family of compounds provides a solution to reducing the resistance to compounds stabilizing the microtubules, in particular of the resistance to paclitaxel. This family also provided new treatments targeting LKB1-deficient cells.

One aim of the family of compounds was to provide compounds for use in a pharmaceutical treatment, notably against a cancer, that could alleviate the above technical limitations, to reduce the resistance to compounds stabilizing the microtubules, in particular the resistance to paclitaxel and to provide compounds being selectively cytotoxic against LKB1-deficient cells.

Chemotherapy agents are still widely used as 1st and 2nd line therapies, although new effective therapeutic approaches against cancer (kinase inhibitors, immune checkpoint inhibitors, etc.) have emerged in recent years. Paclitaxel (PTX) or Taxol^{®} belongs to the taxanes family. This microtubule-targeting agent is considered the world's leading chemotherapeutic agent.

However, its side effects may limit its use to the maximum effective therapeutic dose. Chemotherapy-induced peripheral neuropathy (CIPN) is a common and frequent adverse effect. Physicians are faced with the problem and have no treatment to prevent CIPN. CIPN often persist after chemotherapy and can have a long-term impact on the patient's quality of life.

CIPN is an adverse effect of not only taxanes but also of many chemotherapy agents such as vinca alkaloids, platinum salts, etc., affecting several million patients worldwide every year. CIPN is characterized by altered sensations in the hands and feet, with tingling and burning, or numbness and decreased sensation to touch. Pain symptoms generally appear at the beginning of treatment cycles, while numbness and tingling develop later and can last for years after treatment has stopped.

CIPN has a major impact on several levels. On cancer treatment, as their occurrence leads practitioners to reduce doses of therapeutic agents, thus compromising patients' chances of recovery. On patients' quality of life, not only during treatment but also afterwards, as symptoms persist chronically in half of all patients, and may even prevent them from returning to work. The economic consequences of CIPN are also considerable for the healthcare system.

With the increasing number of cancer survivors due to improved patient's management, CIPN is a growing concern as it negatively and often permanently affects the quality of life of many patients. There are a few symptomatic treatments for neuropathic pain, all of them of questionable efficacy. Duloxetine, a serotonin and norepinephrine reuptake inhibitor approved for the treatment of major depressive disorders, is the only drug recommended by ASCO; however, not all patients benefit from duloxetine (for example, its analgesic efficacy is better against platinum derivatives than against taxanes). Moreover, several undesirable side-effects have been reported for duloxetine such as sleeping difficulties, headaches, etc. Regarding prevention and treatment of CIPN, there is currently no FDA, nor EMA -approved drugs. One of the main obstacles to the development of such neuroprotective drugs is the heterogeneity of the mechanisms - changes in ion channels (sodium, potassium, and calcium), transient receptor potential channels, mitochondrial dysfunction, and immune cell interactions - by which chemotherapeutic agents produce toxicity. Several products are currently undergoing clinical trials- such as those developed by AlgoTx (a topical analgesic hydrogel that inhibits nociceptive sodium channels), by Osmol Therapeutics (intracellular calcium modulation), Sonnet biotherapeutics (recombinant human Interleukin-6), Egetis-Pledpharma (Pledox, an agent acting on oxidative stress, which clinical development has been arrested)- demonstrating the attractiveness of the indication.

There is therefore a need for a compound, or a family of compound that could act as neuroprotectant, alone or in combination with chemotherapeutic treatment.

### DETAILLED DESCRIPTION OF THE INVENTION

In a first aspect, the invention relates to a compound of formula (I) for use as a neuroprotectant, preferably by preventing peripheral neurons from degenerating after chemotherapeutic treatment.

In addition to potentiating the effect of PTX and other taxanes, the inventors have observed that the compounds of formula (I) also exert a neuroprotective effect, preventing peripheral neurons from degenerating after chemotherapy. This neuroprotective action has been validated in vitro, on cultured neurons from dorsal root ganglia (DRG) (Figures 1 and 2) and in vivo on a rat model of neuropathy (Figure 4).

This opens a wide range of application since neuroprotection does not only concern neuropathies resulting from treatment with PTX and taxanes, but also those resulting from treatment with platinum salts such as cisplatin.

Surprisingly, it was discovered by present inventors that a compound of formula (I) of the invention not only potentiates the effect of a compound stabilizing cell microtubules, in particular the effect of paclitaxel, without significantly increasing its toxicity as described in WO2021069736, but also act as neuroprotectants.

It has thus now been observed that the compounds of formula (I) disclosed in WO2021069736 have a dual activity: a synergistic anti-tumor effect with taxanes, reducing doses of these toxic compounds, and a neuroprotective effect.

The inventors have also demonstrated that this neuroprotective effect is independent of its synergistic effect with taxanes on microtubules, observed in tumors, and results not from tubulin targeting but from activation of a metabolic enzyme: nicotinamide phosphoribosyl transferase (NAMPT), most probably. NAMPT is a key enzyme in the nicotinamide adenine dinucleotide (NAD) salvage pathway, playing a central role in maintaining the cellular NAD pool. Numerous studies indicate that NAD is a central modulator controlling the health of damaged or diseased neurons (Verdin, Science, 2015, doi:10.1126/science.aac4854).

The neuroprotective action, independent of its effect on microtubules, would represent the therapeutic solution to CIPN and would therefore be an important differentiating factor answering an important unmet medical need.

Moreover, to our knowledge, there are no drug with such a double mechanism: synergizing with taxanes, enabling dosing regimens reduction, and preventing one of their most incapacitating side effects.

This opens the compounds of formula (I) to a great variety of application, and they can thus be used alone or in combination with a great variety of chemotherapeutic drugs and therapeutic drugs for their neuroprotective effect, preferably by preventing peripheral neurons from degenerating after chemotherapy.

On another aspect, the present invention is the compound of formula (I) for use in the NAD production in patients. The compound for use according to the invention may find applications in the treatment of disorders with reduced NAD metabolism such as:
- central and peripheral neurodegenerative diseases (Alzheimer's, Parkinson's, Huntington's, Amyotrophic Lateral Sclerosis (ALS), Guillain-Barré syndrome);
- neuronal trauma, including noise-induced hearing loss (Brown KD, et al. Activation of SIRT3 by the NAD+ precursor nicotinamide riboside protects from noise-induced hearing loss. Cell Metab. 2014 Dec 2;20(6):1059-68. doi: 10.1016/j.cmet.2014.11.003);
- metabolic diseases such as diabetes or obesity;
- organ damage/failure (liver, kidney, heart);
- certain muscular diseases (Duchene disease);
- ageing;
- metabolic defects affecting sperm mobility;
- the oxidative damage during virus infection.

Another object of the present invention is thus the compound of formula (I) for use in the treatment of disorders with reduced NAD metabolism. The disorders with reduced NAD metabolism may be chosen from the following:
- central and peripheral neurodegenerative diseases such as Alzheimer's disease, Parkinson's disease, Huntington's disease, Amyotrophic Lateral Sclerosis disease;
- neuronal trauma, including noise-induced hearing loss;
- metabolic diseases such as diabetes or obesity;
- organ damage/failure such as liver damage/failure, kidney damage/failure or heart damage/failure;
- certain muscular diseases such as Duchene disease;
- ageing (the effects of ageing resulting from a fall in NAD);
- metabolic defects affecting sperm mobility;
- the oxidative damage during virus infection.

The present invention thus concerns a compound of formula (I): 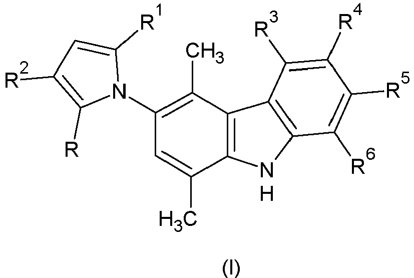 wherein R, R¹ and R² are independently selected form the group consisting of:
- a hydrogen atom,
- a halogen atom,
- an alkyl group, linear, cyclic or branched, saturated or unsaturated, possibly substituted, comprising from 1 to 10 carbon atoms,
- an acyl group comprising from 1 to 10 carbon atoms,
- a carboxyl group,
- an amido group comprising from 1 to 10 carbon atoms, and
- an imino group, possibly substituted by an alkyl group, linear, cyclic or branched, saturated or unsaturated, and
wherein R³, R⁴, R⁵ and R⁶ are independently selected form the group consisting of:
- a hydrogen atom,
- a halogen atom,
- a hydroxyl group,
- an alkyl group, linear, cyclic or branched, saturated or unsaturated, possibly substituted, comprising from 1 to 10 carbon atoms,
- an alkoxy group comprising from 1 to 10 carbon atoms,
- an acyl group comprising from 1 to 10 carbon atoms,
- a carbonate group from 1 to 10 carbon atoms,
- a carboxyl group, and
- a cyano group,
for use as a neuroprotectant, preferably by preventing peripheral neurons from degenerating after chemotherapy.

The present invention thus concerns a compound of formula (I): 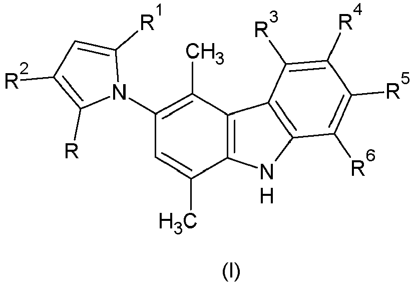 wherein R, R¹ and R² are independently selected form the group consisting of:
- a hydrogen atom,
- a halogen atom,
- an alkyl group, linear, cyclic or branched, saturated or unsaturated, possibly substituted, comprising from 1 to 10 carbon atoms,
- an acyl group comprising from 1 to 10 carbon atoms,
- a carboxyl group,
- an amido group comprising from 1 to 10 carbon atoms, and
- an imino group, possibly substituted by an alkyl group, linear, cyclic or branched, saturated or unsaturated, and
wherein R³, R⁴, R⁵ and R⁶ are independently selected form the group consisting of:
- a hydrogen atom,
- a halogen atom,
- a hydroxyl group,
- an alkyl group, linear, cyclic or branched, saturated or unsaturated, possibly substituted, comprising from 1 to 10 carbon atoms,
- an alkoxy group comprising from 1 to 10 carbon atoms,
- an acyl group comprising from 1 to 10 carbon atoms,
- a carbonate group from 1 to 10 carbon atoms,
- a carboxyl group, and
- a cyano group,
for use in the treatment of defects observed in disorders with a reduced NAD metabolism. The disorders with reduced NAD metabolism may be chosen from the following:
- central and peripheral neurodegenerative diseases such as Alzheimer's disease, Parkinson's disease, Huntington's disease, Amyotrophic Lateral Sclerosis disease;
- neuronal trauma, including noise-induced hearing loss;
- metabolic diseases such as diabetes or obesity;
- organ damage/failure such as liver damage/failure, kidney damage/failure or heart damage/failure;
- certain muscular diseases such as Duchene disease;
- ageing (the ageing resulting from a fall in NAD);
- metabolic defects affecting sperm mobility;
- the oxidative damage during virus infection.

It is meant by neuroprotectant an agent that protects against neuronal degeneration secondary to chemotherapeutic treatment, trauma, metabolic damage, neurological damage (ALS, Alzheimer's, etc.). It may be preventing peripheral neurons from degenerating after chemotherapy.

The compound of formula (I) for use according to the invention may also be in the form of its tautomeric, racemic, enantiomeric or polymorphic forms or pharmaceuticallyacceptable salts.

The compound of formula (I) for use according to the invention may also be in the form of co-crystal, said co-crystal comprising the compound of formula (I) and a co-former. The co-former may be chosen from nicotinamide, isonicotinamide, nicotinamide riboside, nicotinamide mononucleotide, ascorbic acid, syringic acid, gallic acid and caffeine.

The invention refers to a "substituted" group or moiety, which is known to mean that at least one hydrogen radical of said group or moiety is replaced with an atom or a group of atoms called substituent.

Examples of preferred substituents are halogen (chloro, iodo, bromo, or fluoro); alkyl; alkenyl; alkynyl; hydroxy; alkoxy; nitro; thiol; thioether; imine; cyano; amido; phosphonato; phosphine; carboxyl; thiocarbonyl; sulfonyl; sulfonamide; ketone; aldehyde; ester; oxygen (-O); haloalkyl (e.g., trifluoromethyl); cycloalkyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic (e.g., pyrrolidinyl, piperidinyl, piperazinyl, morpholinyl, or thiazinyl), monocyclic or fused or non-fused polycyclic aryl or heteroaryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); CO₂CH₃; CONH₂; OCH₂CONH₂; NH₂; SO₂NH₂; OCHF₂; CF₃; OCF₃; and such moieties may also be optionally substituted by a fused-ring structure or bridge, for example -OCH₂O-. These substituents may optionally be further substituted with a substituent selected from such groups.

The invention refers to an "acyl" group, which is known as a moiety derived by the removal of the hydroxyl group from a carboxylic acid. Examples of acyl groups are aldehydes, ketones, esters, amides or acyl chlorides.

Preferably, in the compound of formula (I) for use according to the invention, R, R¹ and R² are independently selected from the group consisting of:
- a hydrogen atom,
- a halogen atom,
- an alkyl group of formula -CₙH₂ₙ₊₁₋ₓXₓ, X being selected from the group consisting of a hydrogen atom, a halogen atom, a hydroxyl group, and n being an integer between 1 and 10 and x being the number of X present in the alkyl group, and preferably x being 1 and X being in terminal position in the alkyl group,
- an alkyl group bearing an amino (primary, secondary, or tertiary),
- an alkyl group bearing an ester, the ester being preferably an ester of a protected (e.g. Boc, Fmoc or Cbz protected) amino acid such as lysine, valine, tyrosine, cysteine, glutamic acid, an ester of a diacid, such as succinic acid or an ester of acid bearing a polyethylene glycol, having from 4 to 50 repeating units,
- an alkyl group bearing piperidine or a dipiperidine linked via a carbamate,
- an acyl group of formula -C(O)ORₐ, Rₐ being a linear and saturated alkyl group comprising from 1 to 9 carbon atoms,
- an acyl group of formula -C(O)H,
- a carboxyl group (-COOH),
- an amido group of formula -C(O)NR_{b}, R_{b} being a linear and saturated alkyl group comprising from 1 to 9 carbon atoms, and
- an imino group of formula -C=N-R_{c}, R_{c} being selected from the group consisting of a hydrogen atom or an alkyl group, linear, saturated and comprising from 1 to 10 carbon atoms, or a phenyl group, and
R³, R⁴, R⁵ and R⁶ are independently selected from the group consisting of:
- a hydrogen atom,
- a halogen atom,
- a hydroxyl group,
- an alkyl group of formula -CₙH₂ₙ₊₁, linear, saturated and n being an integer between 1 and 10,
- an alkoxy group of formula -OR_{d}, R_{d} being a linear and saturated alkyl group comprising from 1 to 9 carbon atoms,
- an acyl group of formula -C(O)ORₐ, Rₐ being a linear and saturated alkyl group comprising from 1 to 9 carbon atoms,
- an acyl group of formula -C(O)Rₑ, Rₑ being a hydrogen atom or an alkyl group, linear, cyclic or branched, saturated or unsaturated, possibly substituted, comprising from 1 to 10 carbon atoms,
- a carbonate group of formula -OC(O)OR_{f}, R_{f} being a linear and saturated alkyl group comprising from 1 to 9 carbon atoms,
- a carboxyl group (-COOH), and
- a cyano group (-CN).

More preferably, in the compound of formula (I) for use according to the invention, R and R¹ are independently selected from the group consisting of:
- a hydrogen atom,
- a halogen atom,
- an alkyl group, linear, saturated and comprising from 1 to 10 carbon atoms,

R² is selected from the group consisting of:
   - a hydrogen atom,
   - a halogen atom,
   - an alkyl group of formula -CₙH₂ₙ₊₁₋ₓXₓ, X being selected from the group consisting of a hydrogen atom, a halogen atom, a hydroxyl group, and n being an integer between 1 and 10 and x being the number of X present in the alkyl group, and preferably x being 1 and X being in terminal position in the alkyl group,
   - an alkyl group bearing an amino (primary, secondary, or tertiary),
   - an alkyl group bearing an ester, the ester being preferably an ester of a protected (e.g. Boc, Fmoc or Cbz protected) amino acid such as lysine, valine, tyrosine, cysteine, glutamic acid, an ester of a diacid, such as succinic acid or an ester of acid bearing a polyethylene glycol, having from 4 to 50 repeating units,
   - an alkyl group bearing piperidine or a dipiperidine linked via a carbamate,
   - an acyl group of formula -C(O)ORₐ, Rₐ being a linear and saturated alkyl group comprising from 1 to 9 carbon atoms,
   - an acyl group of formula -C(O)H,
   - a carboxyl group (-COOH),
   - an amido group of formula -C(O)NR_{b}, R_{b} being a linear and saturated alkyl group comprising from 1 to 9 carbon atoms, and
   - an imino group of formula -C=N-R_{c}, R_{c} being selected from the group consisting of a hydrogen atom or an alkyl group, linear, saturated and comprising from 1 to 10 carbon atoms, or a phenyl group,
R³, R⁵ and R⁶ are independently selected from the group consisting of:
   - a hydrogen atom,
   - a halogen atom,
   - an alkyl group of formula -CₙH₂ₙ₊₁, linear, saturated and n being an integer between 1 and 10, and
R⁴ is selected from the group consisting of:
   - a hydrogen atom,
   - a halogen atom,
   - a hydroxyl group,
   - an alkyl group of formula -CₙH₂ₙ₊₁, linear, saturated and n being an integer between 1 and 10,
   - an alkoxy group of formula -OR_{d}, R_{d} being a linear and saturated alkyl group comprising from 1 to 9 carbon atoms,
   - an acyl group of formula -C(O)ORₐ, Rₐ being a linear and saturated alkyl group comprising from 1 to 9 carbon atoms,
   - an acyl group of formula -C(O)Rₑ, Rₑ being a hydrogen atom or an alkyl group, linear, cyclic or branched, saturated or unsaturated, possibly substituted, comprising from 1 to 10 carbon atoms,
   - a carbonate group of formula -OC(O)OR_{f}, R_{f} being a linear and saturated alkyl group comprising from 1 to 9 carbon atoms,
   - a carboxyl group (-COOH), and
   - a cyano group (-CN).

Even more preferably, in the compound of formula (I) for use according to the invention, R and R¹ are independently selected from the group consisting of a hydrogen atom, a chloride atom and a methyl group,
R² is selected from the group consisting of a hydrogen atom, a methyl group, a -COH group, an alkyl group of formula -CH₂X, X being selected from the group consisting of a chloride atom, a bromide atom, a iodine atom and a hydroxyl group, an acyl group of formula -C(O)ORₐ, Rₐ being a methyl group or an ethyl group, an acyl group of formula -C(O)H, a carboxyl group (-COOH), an amido group of formula -C(O)NR_{b}, R_{b} being a methyl group or an ethyl group, an imino group of formula -C=N-R_{c}, R_{c} being a phenyl group, -CH₂Y, Y being selected from the group consisting of an amino group of formula -NR_{z}R_{z}, R_{z} independently representing H or a C1 to C3 alkyl group, an ester, the ester being preferably an ester of a protected (e.g. Boc, Fmoc or Cbz protected) amino acid such as lysine, valine, tyrosine, cysteine, glutamic acid, an ester of a diacid, such as succinic acid or an ester of acid bearing a polyethylene glycol, having from 4 to 50 repeating units, or a piperidine or a dipiperidine linked via a carbamate,
R³, R⁵ and R⁶ are independently selected from the group consisting of a hydrogen atom, a chloride atom, a methyl group and an ethyl group, and
R⁴ is selected from the group consisting of a hydrogen atom, a methyl group, a hydroxyle group, a chloride atom, a bromide atom, a fluoride atom, a iodine atom, an alkoxy group of formula -OR_{d}, R_{d} being a methyl group or an ethyl group, an acyl group of formula -C(O)ORₐ, Rₐ being a methyl group or an ethyl group, an acyl group of formula -C(O)Rₑ, Rₑ being a methyl group, an ethyl group, a linear propyl group, a linear pentyl group, - CH₂CH₂-cyclopentyl group and a (*para*-methyl)phenyl group, a carbonate group of formula -OC(O)OR_{f}, R_{f} being a methyl group or an ethyl group, a carboxyl group (-COOH), and a cyano group (-CN).

Advantageously, in the compound of formula (I) for use according to the invention, R and R¹ are independently selected from the group consisting of a hydrogen atom and a methyl group,
R² is selected from the group consisting of a hydrogen atom, a -COH group, a - CH₂OH group, a -CH₂NHC₂H₅ group, a -CH₂N(C₂H₅)₂ group, a -CH₂O-ValBoc group, a - CH₂O-(Boc)LysBoc group, a -CH₂OOC(CH₂)₂COOH group, a -CH₂OCO(PEG)-OCH₃ group and a -CH₂OCO(NC₅H₉)NC₅H₁₀ group,
R³, R⁵ and R⁶ are independently selected from the group consisting of a hydrogen atom, a chloride atom and an ethyl group, and
R⁴ is selected from the group consisting of a hydrogen atom, a hydroxyl group, a chloride atom, a bromide atom, a fluoride atom, a carbonate group of formula -OC(O)OR_{f}, R_{f} being an ethyl group and an acyl group of formula -C(O)ORₐ, Rₐ being a methyl group.

According to an embodiment, the compound of formula (I) for use according to the invention is characterized in that at least three of the substituents selected from the group consisting of R³, R⁴, R⁵ and R⁶ are a hydrogen atom.

According to an embodiment, the compound of formula (I) comprises at least one of the substituents selected from the group consisting of R, R¹ and R² is a hydrogen atom.

According to an embodiment, the compound of formula (I) comprises at least two of the substituents selected from the group consisting of R, R¹ and R² are a hydrogen atom.

According to an embodiment, R = R¹ = R² = H.

In a preferred embodiment, the compound of formula (I) is selected from the group consisting of

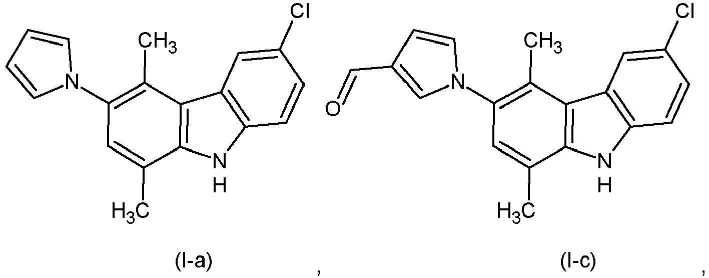

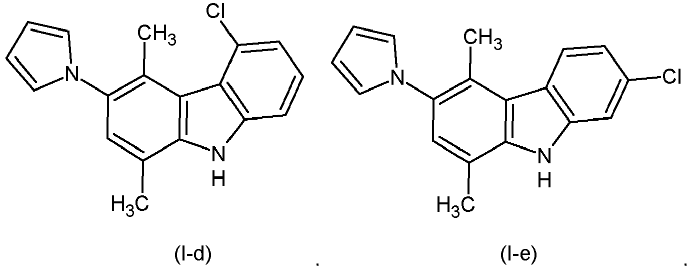

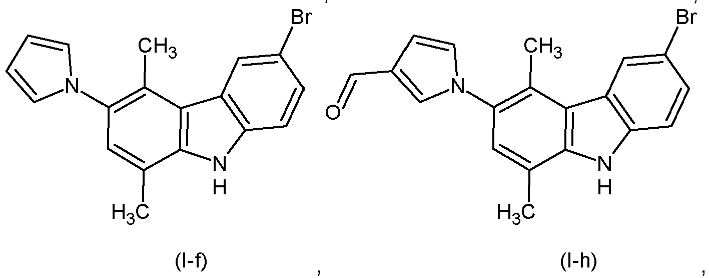

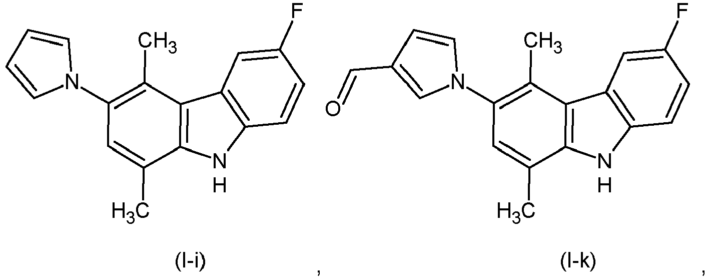

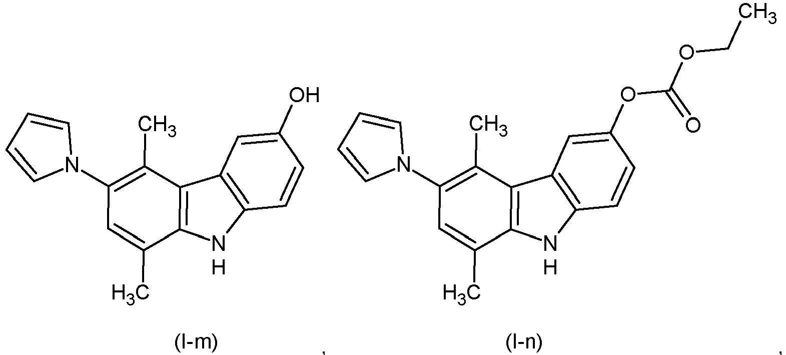

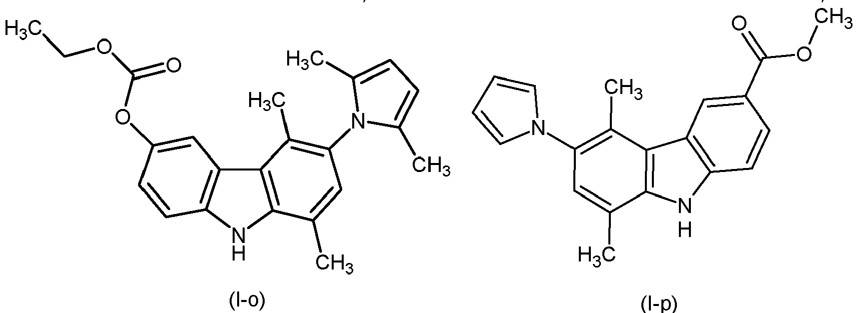

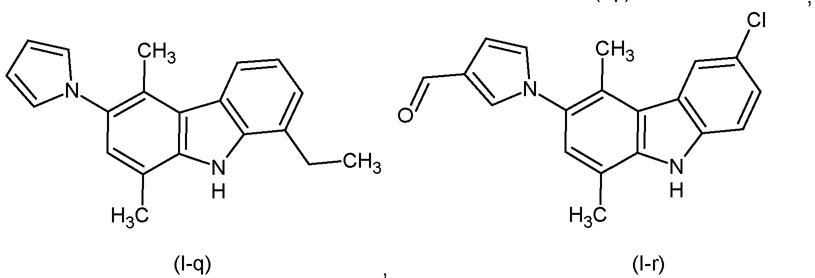

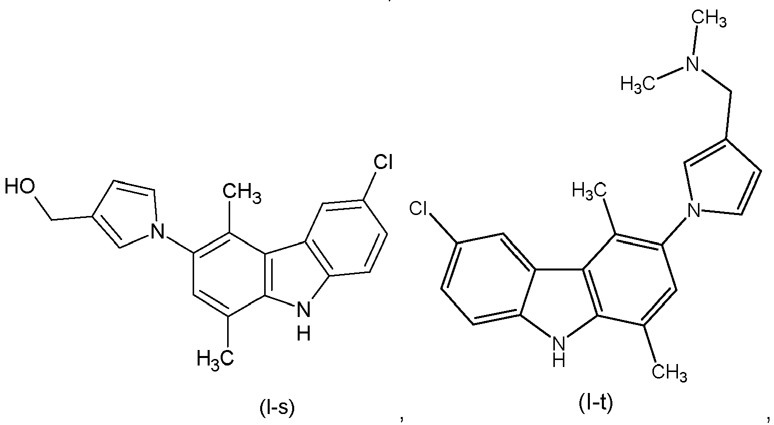

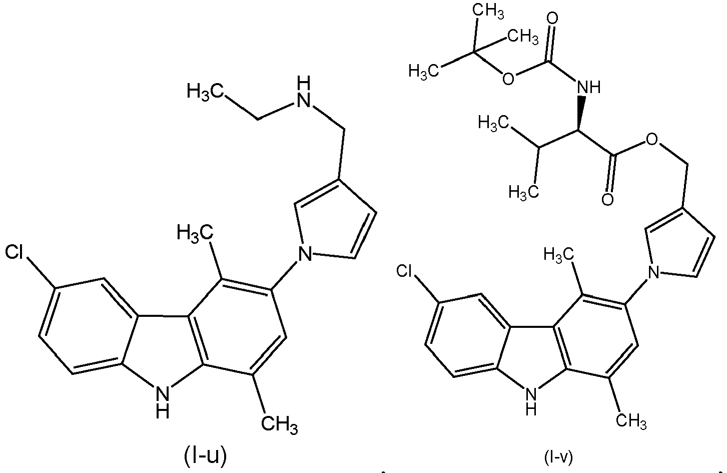

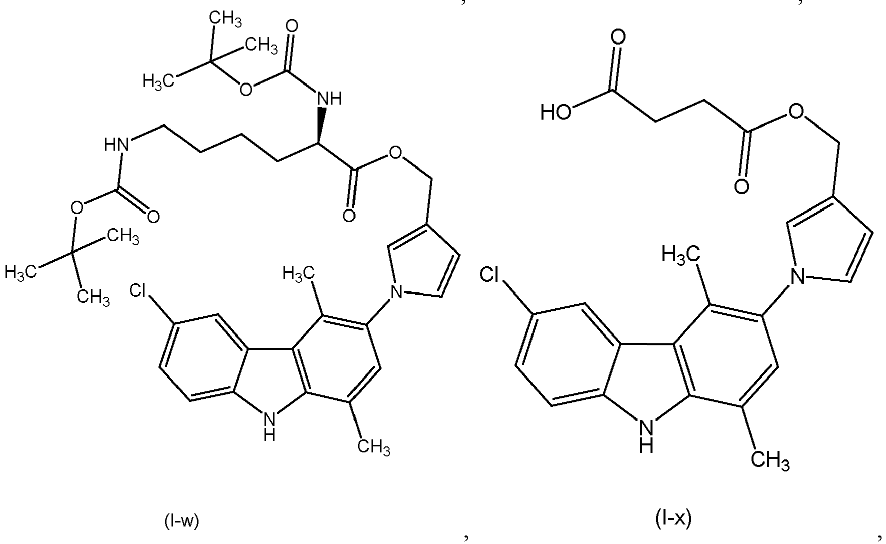

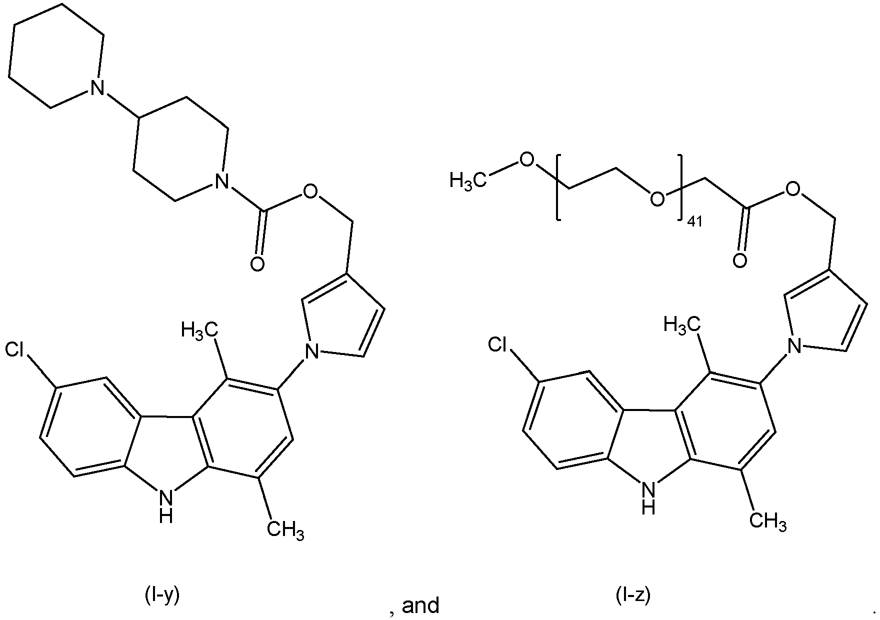

In a preferred embodiment, the compound of formula (I) is selected from the group consisting of 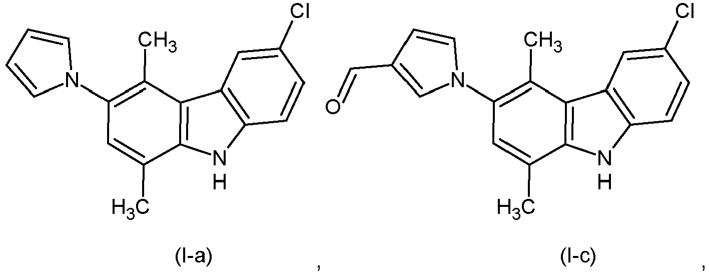 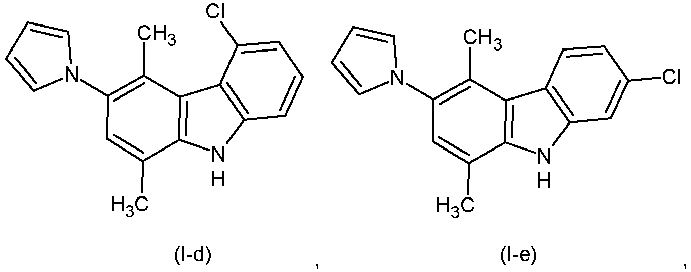 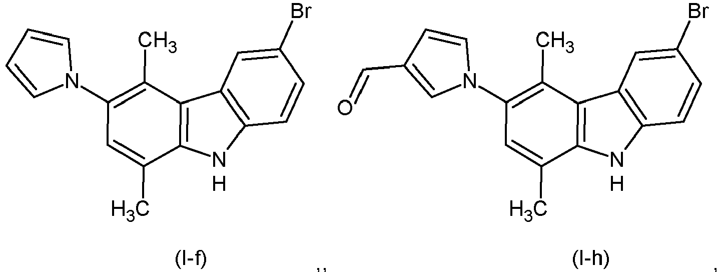 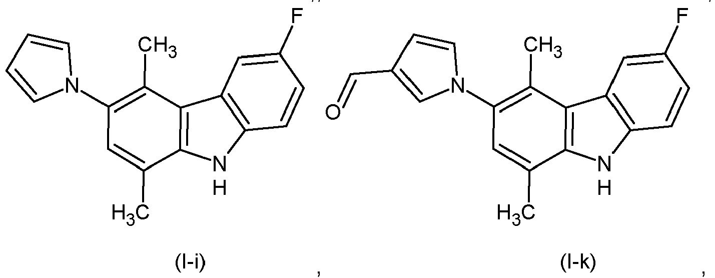 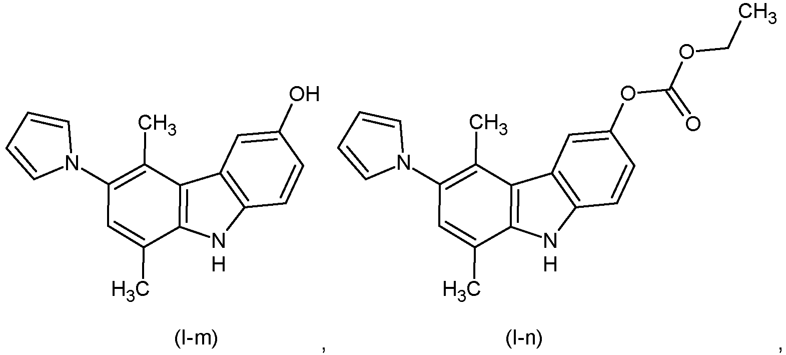 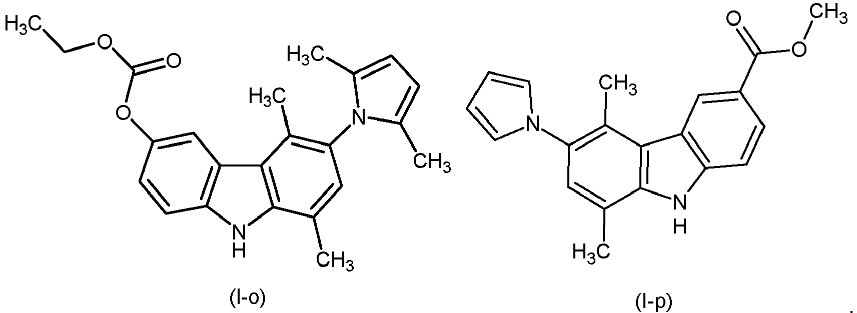 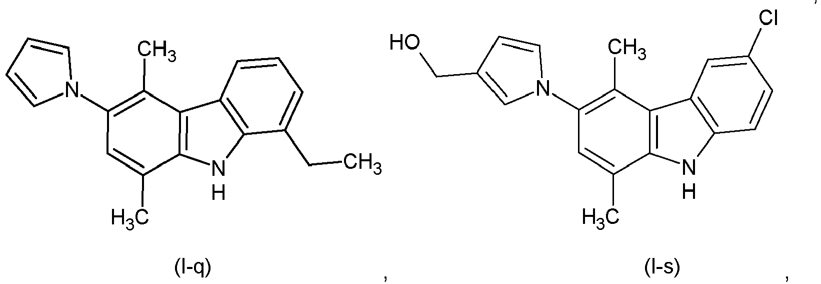 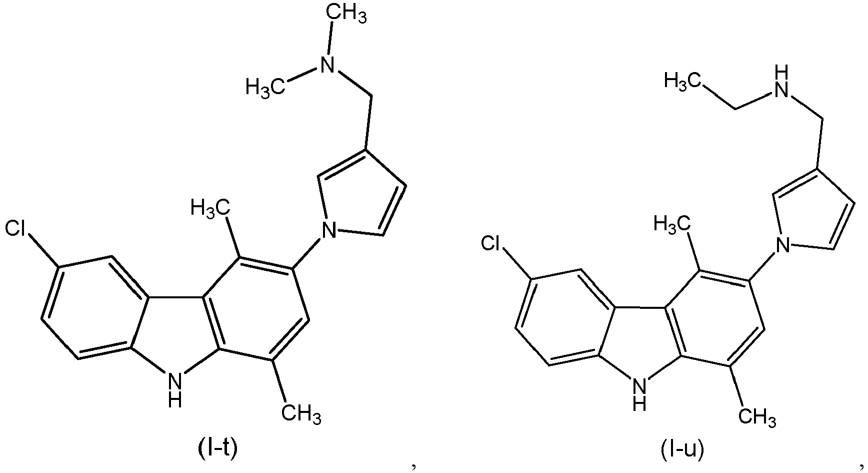 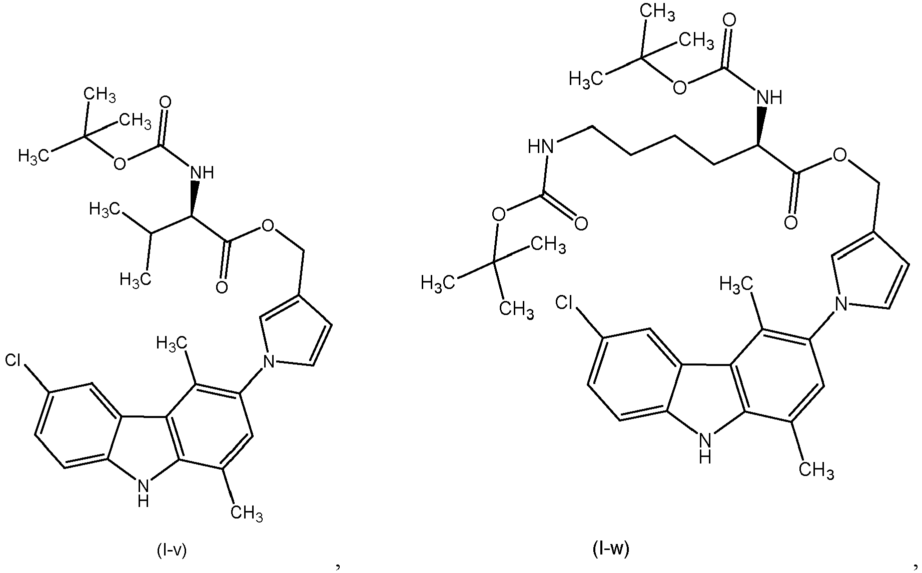 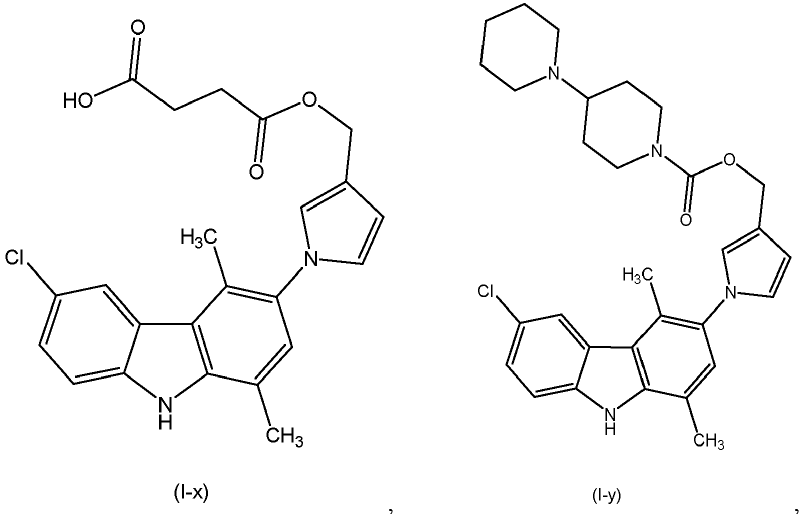 and

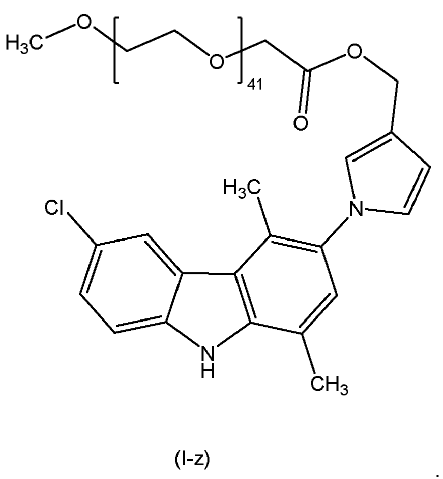

Preferably, a compound of formula (I) is selected from the group consisting of a compound of formula (I-a), a compound of formula (I-f), a compound of formula (I-h), a compound of formula (I-e), a compound of formula (I-m), a compound of formula (I-n), a compound of formula (I-r), a compound of formula (I-s), a compound of formula (I-t), a compound of formula (I-u), a compound of formula (I-w), a compound of formula (I-x), a compound of formula (I-y), a compound of formula (I-z), and any combination thereof
Preferably, a compound of formula (I) is selected from the group consisting of a compound of formula (I-a), a compound of formula (I-f), a compound of formula (I-t), a compound of formula (I-u), and any combination thereof.

Preferably, a compound of formula (I) is selected from the group consisting of a compound of formula (I-a), a compound of formula (I-f), a compound of formula (I-t), a compound of formula (I-u), and any combination thereof.

Preferably, a compound of formula (I) is selected from the group consisting of a compound of formula (I-a), a compound of formula (I-f), a compound of formula (I-r), a compound of formula (I-s), a compound of formula (I-t), a compound of formula (I-u), and any combination thereof.

More preferably, a compound of formula (I) is selected from the group consisting of a compound of formula (I-a), a compound of formula (I-r), a compound of formula (I-s), a compound of formula (I-f), a compound of formula (I-t), a compound of formula (I-u), and any combination thereof.

Advantageously, the compound of formula (I) is a compound of formula (I-a).

Advantageously, the compound of formula (I) is a compound of formula (I-r), a compound of formula (I-s), a compound of formula (I-f), a compound of formula (I-w), a compound of formula (I-x), a compound of formula (I-y), a compound of formula (I-z), and any combination thereof.

The present invention also concerns a pharmaceutical composition for use as neuroprotectant, comprising at least one compound of formula (I) according to the invention and at least one pharmaceutically acceptable excipient. In the pharmaceutical composition for use according to the invention, the compound of formula (I) may also be in the form of co-crystal, said co-crystal comprising the compound of formula (I) and a co-former. The co-former may be chosen from nicotinamide, isonicotinamide, nicotinamide riboside, nicotinamide mononucleotide, ascorbic acid, syringic acid, gallic acid and caffeine.

The present invention also concerns a pharmaceutical composition for use in the treatment of defects observed in disorders with a reduced NAD metabolism, comprising at least one compound of formula (I) according to the invention and at least one pharmaceutically acceptable excipient. In the pharmaceutical composition for use according to the invention, the compound of formula (I) may also be in the form of co-crystal, said co-crystal comprising the compound of formula (I) and a co-former. The co-former may be chosen from nicotinamide, isonicotinamide, nicotinamide riboside, nicotinamide mononucleotide, ascorbic acid, syringic acid, gallic acid and caffeine.

The expression "pharmaceutically acceptable excipient" refers to any diluents, adjuvants or vehicles, such as preserving agents, fillers, disintegrating agents, wetting agents, emulsifying agents, suspending agents, solvents, dispersion media, coatings, antibacterial and antifungal agents, isotonic and absorption delaying agents and the like.

The expression "patient" may refer either to a human patient or to an animal patient.

The pharmaceutical composition of the present invention may be administered by any suitable route, for example, by oral, buccal, inhalation, sublingual, nasal, percutaneous, i.e. transdermal or parenteral (including intravenous, intramuscular, subcutaneous and intracoronary) administration. Therefore, the pharmaceutical composition of the invention can be provided in various forms, such as in the form of hard gelatin capsules, of capsules, of compressed tablets, of suspensions to be taken orally, of lozenges or of injectable solutions, ointments, or in any other form appropriate to the method of administration.

The exact formulation, route of administration, and dosage can be chosen by the individual physician in view of the patient's conditions. Dosage amount and interval of administration can be adjusted individually to provide plasma levels of compound of formula (I) which are sufficient to maintain the preventive or therapeutic effects. The amount of pharmaceutical composition administered therefore depends on the subject being treated, on the subject's weight, the severity of the affliction and the manner of administration.

The invention also relates to a co-crystal comprising one compound of formula (I) as defined in any one of claims 1 to 7 and a co-former, the co-former being preferably chosen from nicotinamide, isonicotinamide, nicotinamide riboside, nicotinamide mononucleotide, ascorbic acid, syringic acid, gallic acid, caffeine.

The invention will be better understood from reading the following non-limiting examples.

### FIGURES

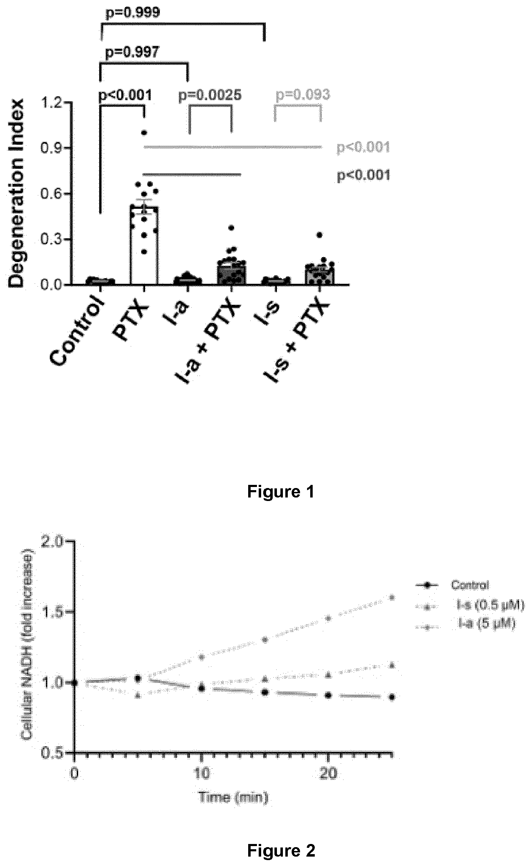

Figure 1 represents the quantitative analysis of the neuroprotective effect of compound I-a and I-s on isolated mouse adult DRG .
Figure 2 represents the kinetics of NADH autofluorescence in HeLa cells after treatment with I-a (5 µM) or I-s (0.5 µM).
As shown on Figure 2, in comparison with controls (dots) a dose-dependent increase in NADH production after I-a (lozenges) and I-s (triangles) treatment was measured during the 25-minute analysis. This clearly indicates an activation of the pathways leading to NADH production.

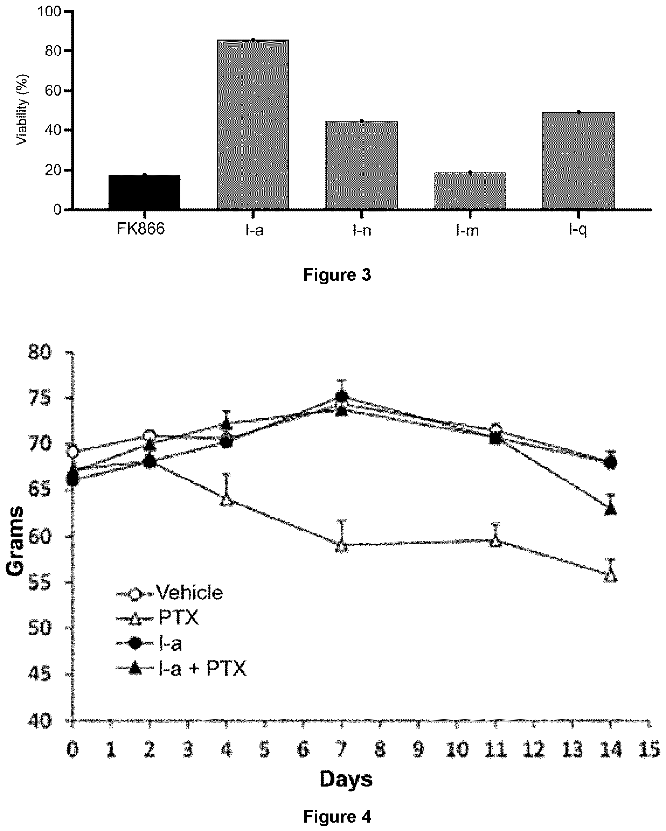

Figure 3 represents the effect of compounds I-a, I-f, I-m, I-n, I-q and the comparative example on the cytotoxicity mediated by NAMPT inhibitor FK866.
   HeLa cells were seeded in microplates and incubated with 3.5 nM FK866 without (black) or in the presence of 6 µM of I-a and different analogues (grey), as indicated, for 72 hours. A viability assay (prestoblue assay) was then conducted. While FK866 induced cell death of over 80% of cells, leaving only 20% alive cells (Y-axis), co-incubation of this inhibitor with compounds I-a, I-n and I-q significantly reduced cell death. Compound I-m had no detectable protective effect. These results show that compounds I-a, I-f, I-m, I-n and I-q can relieve the cytotoxicity of the NAMPT inhibitor FK866, strongly suggesting that activation of NAMPT is involved in the protective activity of these compounds. Of note, the comparative example does not reduce FK866-induced cell death.
Figure 4 represents the effect of compound I-a on tactile sensitivity in a rat model of paclitaxel-induced neuropathy. Behavioral assessments were performed on 5-week-old male Sprague-Dawley rats before (basal values) and at days 4, 7, 11 and 14 after the beginning of the drug injections (Compound I-a 50 mg/kg, paclitaxel (PTX) 4 mg/kg, Compound I-a 50 mg/kg + PTX 4 mg/kg). Control animals (Vehicle) received vehicle injections. The results are presented by mean ± standard deviation (n = 6 per group). Tactile nociceptive thresholds were assessed using an electronic von Frey test (J. Ferrier, F. Marchand, D. Balayssac, Assessment of mechanical allodynia in rats using the electronic von frey test, BIO-Protoc., 6 (2016), 10.21769/BioProtoc.1933). Rats were placed individually in plastic compartments on an elevated wire floor and left for habituation for 15 min before each experiment. The von Frey apparatus, consisting of a plastic tip fitted in a hand-held force transducer, was applied perpendicularly to the animal's right hind paw from below and the force applied was gradually increased until paw withdrawal. The maximum force applied (expressed in grams) to induce paw withdrawal was recorded automatically.

As shown on the figure, while rats treated with PTX (white triangle) developed progressively tactile hypersensitivity with a decrease of thresholds in comparison to basal value at day 0, rats treated with compound I-a, alone (black dots) or in combination with PTX (black triangles), presented tactile thresholds not different from those of the vehicle (white dots), clearly demonstrating that compound I-a has no effect in itself and prevents the hypersensitivity induced by PTX.

### EXAMPLES

### Example 1: Synthesis of compounds of formula (I)

The following compounds of formula (I) were synthesized according to the procedures described in WO2021069736.

The following compounds were prepared:

| **Références composés** | **Structures** |
|---|---|
| (I-a) | 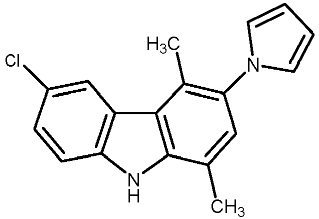 |
| (I-p) | 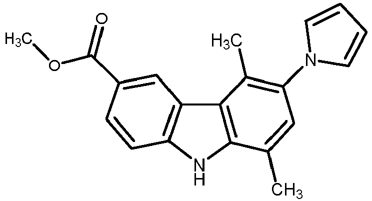 |
| (I-i) | 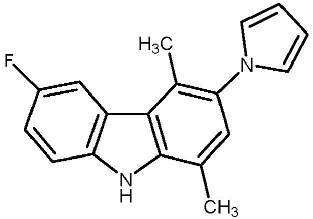 |
| (I-f) | 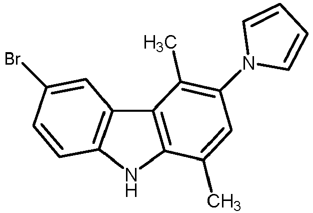 |
| (I-m) | 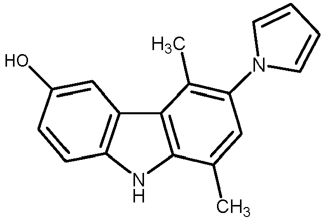 |
| (I-n) | 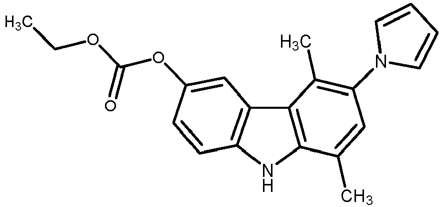 |
| (I-q) | 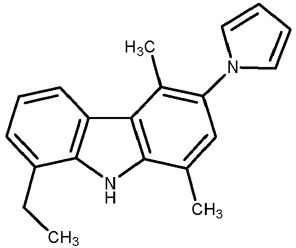 |
| (I-e) | 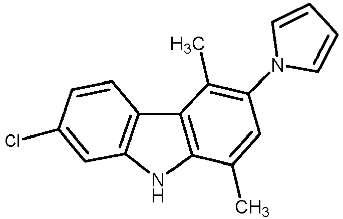 |
| (I-d) | 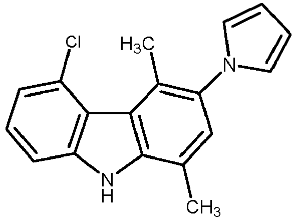 |
| (I-g) | 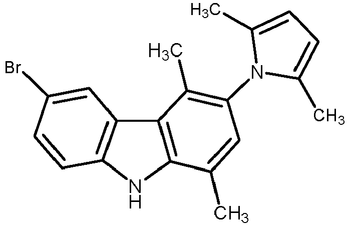 |
| (I-o) | 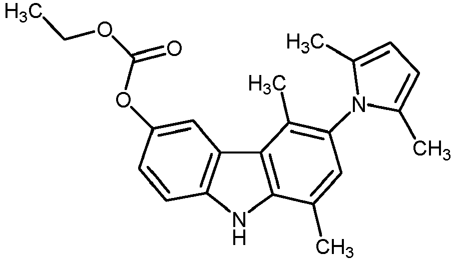 |
| (I-k) | 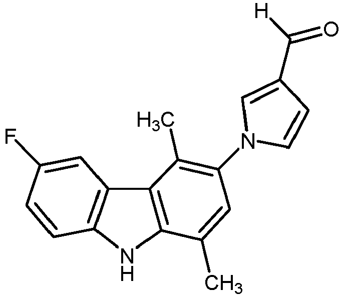 |
| (I-h) | 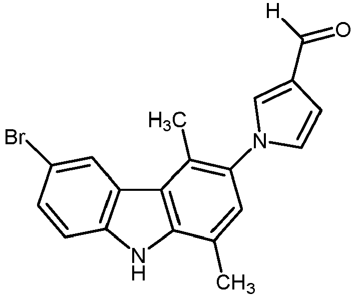 |
| (I-s) | 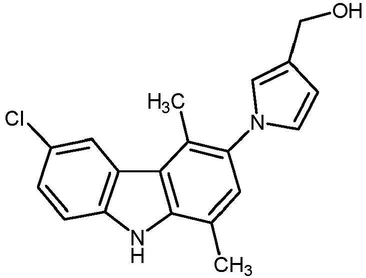 |
| (I-t) | 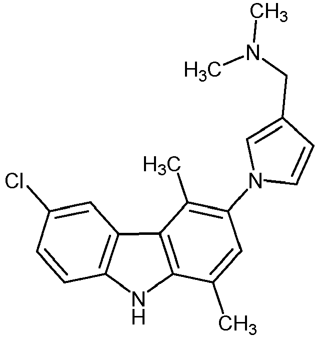 |
| (I-u) | 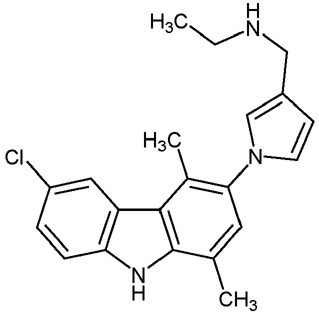 |
| (I-v) | 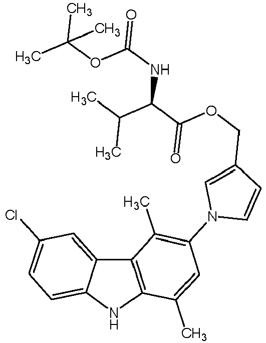 |
| (I-w) | 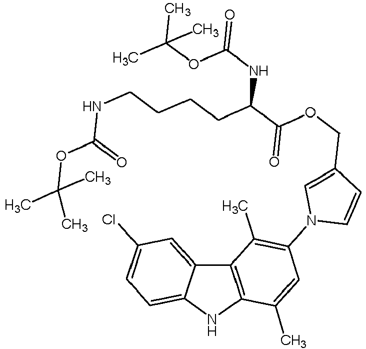 |
| (I-x) | 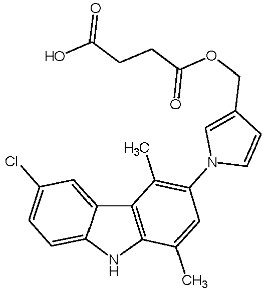 |
| (I-y) | 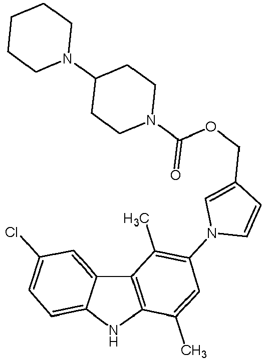 |
| (I-z) | 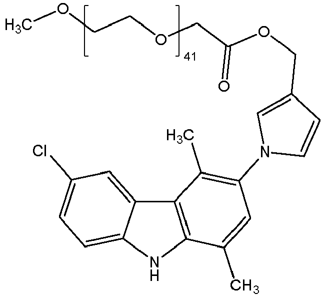 |
| (I-y) (HCl) | 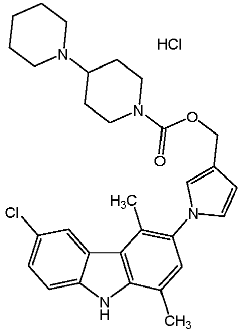 |
| Comparative example | 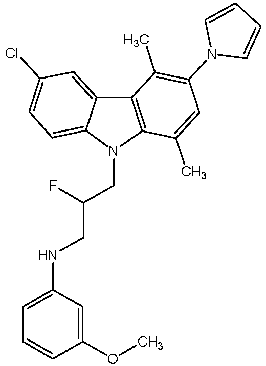 |

### Synthesis of compounds (I-t), (I-u),

Dichloro[1,3-dihydro-1,3-bis(1-methylethyl)-2*H*-imidazol-2-ylidene][(1,2,3,4,5-η)-1,2,3,4,5-pentamethyl-2,4-cyclopentadien-1-yl]iridium (0.5-1.0 mol %), K₂CO₃ (5 mol %), compound *(I-s)* (1.0 mmol) and a 50% aqueous solution of dimethylamine or ethylamine (6.0 mmol) are mixed in a stainless tube. The latter is sealed and the mixture is stirred for 20 hours at 120-130°C. The reaction mixture is then cooled to room temperature and the product is extracted with dichloromethane. The solvent is evaporated to dryness and the crude product is purified through silica gel chromatography.

### Synthesis of compounds (I-v), (I-w), (I-x), (I-y), (I-z)

To one equivalent of *(I-s)*in pyridine at RT was added 1.1 equivalent of acid chloride and one crystal of 4-(dimethylamino)pyridine. After 12h, the reaction was quenched with saturated NH₄Cl, extracted with EtOAc and dried with Na₂SO₄ to give the crude esters. The latter were purified through silica gel chromatography.

| Compound | T_{fusion} (°C) | IR (KBr) (cm⁻¹) | ¹H NMR and MS (ESI) |
|---|---|---|---|
| (I-t) | 93 | 3111, 1512, 1450, 1302. | ¹H-NMR (CDCl₃, 400Mz): 8.38 (s, 1H), 8.10 (s, 1H), 7.46 (d, *J*= 8.56Hz, 1H), 7.39 (dd, *J*= 8.56, 1.37Hz, 1H), 7.15 (s, 1H), 6.86 (s, 1H), 6.76 (s, 1H), 6.31 (s, 1H), 3.70 (s, 2H), 2.54-2.49 (m, 12H). |
| | | | MS [M+H]⁺ for C₂₁H₂₃³⁵ClN₃= 352.00. |
| (I-u) | 68 | 3107, 1510, 1448, 1300. | ¹H-NMR (CDCl₃, 400Mz): 8.24 (bs, 1H), 8.13 (d, *J*= 1.40Hz, 1H), 7.43 (dd, *J*=8.68, 0.50Hz, 1H), 7.39 (dd, *J*=8.71, 1.80Hz, 1H), 7.17 (bs, 1H), 6.74 (m, 2H), 6.29 (t, *J*= 2.17Hz, 1H), 3.79 (s, 2H), 2.79 (q, *J*= 7.17Hz, 2H), 2.56 (s, 3H), 2.53 (s, 3H), 1.19 (t, *J*= 7.88Hz, 3H). |
| | | | MS [M+H]⁺ for C₂₁H₂₃³⁵ClN₃ = 352.00 |
| (I-v) | 82 | 3279, 2930, 1670, 1444 | ¹H NMR (600 MHz, CDCl₃) δ 8.36 (s, 1H), 8.12 (d, *J* = 1.9 Hz, 1H), 7.43 (d, *J* = 8.6 Hz, 1H), 7.40 (dd, *J* = 8.6, 1.9 Hz, 1H), 7.15 (s, 1H), 6.82 (bs, 1H), 6.73 (bs, 1H), 6.32 (s, 1H), 5.20 (d, *J* = 12.1 Hz, 1H), 5.10 (m, 2H), 4.28 (dd, *J* = 9.3, 4.6 Hz, 1H), 2.53 (s, 6H), 2.21 - 2.13 (m, 1H), 1.45 (s, 9H), 0.95 (d, *J* = 6.8 Hz, 3H), 0.87 (d, *J* = 6.9 Hz, 3H). |
| | | | MS [M+Na]⁺ for C₂₉H₃₄³⁵ClN₃N₈O₄ = 546.21 |
| (I-w) | 80 | 3344, 2976, 1687, 1510. | ¹H NMR (600 MHz, CDCl₃) δ 8.23 (s, 1H), 8.14 (d, *J* = 1.9 Hz, 1H), 7.44 (d, *J* = 8.6 Hz, 1H), 7.41 (dd, *J* = 8.6, 1.9 Hz, 1H), 7.17 (s, 1H), 6.85 (bs, 1H), 6.76 (t, *J* = 2.5 Hz, 1H), 6.35 - 6.32 (m, 1H), 5.21 - 5.09 (m, 3H), 4.56 (s, 1H), 4.36 - 4.29 (m, 1H), 3.13 - 3.05 (m, 2H), 2.55 (d, *J* = 7.2 Hz, 6H), 1.86 - 1.81 (m, 1H), 1.72 - 1.62 (m, 1H), 1.40 (s, 18H), 1.40 - 1.29 (m, 2H). |
| | | | MS [M+Na]⁺ for C₃₅H₄₅³⁵ClN₄NaO₆= 675.29 |
| (I-x) | 75 | 3371, 1708, 1157. | ¹H NMR (600 MHz, CDCl₃) δ 8.16 - 8.11 (m, 2H), 7.43 (dd, *J* = 8.5, 0.6 Hz, 1H), 7.41 (dd, *J* = 8.6, 1.9 Hz, 1H), 7.18 (s, 1H), 6.85 (t, *J* = 2.0 Hz, 1H), 6.76 (t, *J* = 2.4 Hz, 1H), 6.34 (dd, *J* = 2.7, 1.8 Hz, 1H), 5.13 (s, 2H), 2.71 -2.68 (m, 4H), 2.57 (s, 3H), 2.54 (s, 3H). |
| | | | MS [M+Na]⁺ for C₂₃H₂₁³⁵ClN₂N₈O₄= 447.11 |
| (I-y) | 78 | 3279, 2930, 1670, 1445. | ¹H NMR (600 MHz, MeOD) δ 7.89 (d, *J* = 2.0 Hz, 1H), 7.30 (d, *J* = 8.6 Hz, 1H), 7.17 (dd, *J* = 8.6, 2.0 Hz, 1H), 6.93 (s, 1H), 6.67 (t, *J* = 2.0 Hz, 1H), 6.56 (t, *J* = 2.4 Hz, 1H), 6.10 (dd, *J* = 2.7, 1.7 Hz, 1H), 4.87 (s, 2H), 4.05 - 3.97 (m, 2H), 3.15 (s, 1H), 2.58 (s, 2H), 2.35 (m, 6H), 2.31 (s, 3H), 2.26 (m,1H), 1.66 (m, 2H), 1.40 (p, *J* = 5.7 Hz, 4H), 1.30 - 1.14 (m, 4H), 1.08 (s, 1H). |
| | | | MS [M+H]⁺ for C₃₀H₃₆³⁵ClN₄O₂= 519.25 |
| (I-z) | 80 | 2883, 1732, 1465, 1340. | ¹H NMR (500 MHz, CDCl₃) δ 9.07 (s, 1H), 8.13 (d, *J* = 2.1 Hz, 1H), 7.48 (d, *J* = 8.6 Hz, 1H), 7.39 (dd, *J* = 8.6, 2.0 Hz, 1H), 7.17 (s, 1H), 6.85 (t, *J* = 2.0 Hz, 1H), 6.76 (t, *J* = 2.4 Hz, 1H), 6.34 (t, *J* = 2.2 Hz, 1H), 5.12 (s, 2H), 4.26 - 4.22 (m, 2H), 3.65-3.61 (m, 164H), 3.38 (s, 3H), 2.69 (s, 6H). |
| | | | MS [M+H]⁺ for C₁₀₄H₁₈₆³⁵ClN₂O₄₄= 2201.27 |

### Synthesis of compound (I-y) (HCl):

To absolute EtOH (10 ml) was added [1-(6-chloro-1,4-dimethyl-9H-carbazol-3-yl)pyrrol-3-yl]methyl 4-(1-piperidyl)piperidine-1-carboxylate (0.020 g, 0.038 mmol). The suspension was heated up to 70°C to dissolve the molecule and then allowed to cool down to room temperature. Hydrogen chloride was bubbled through the solution for a short period then the reaction mixture was stirred at room temperature for 15 minutes. The reaction mixture was then degassed with nitrogen for 10 minutes and filtered. The precipitate was washed with Et₂O affording the desired compound in 70% yield.
1H NMR (600 MHz, MeOD) δ 8.12 (s, 1H), 7.53 (d, *J* = 8.6 Hz, 1H), 7.40 (d, *J* = 8.6Hz, 1H), 7.15 (s, 1H), 6.91 (s, 1H), 6.80 (s, 1H), 6.33 (s, 1H), 5.11 (s, 2H), 4.37 (d, *J=* 13.6 Hz, 2H), 3.59 (d, *J* = 13.3 Hz, 1H), 3.50 (s, 1H), 3.39 (m, 1H), 3.12 (t, *J* = 13.2 Hz, 1H), 3.04 (s, 2H), 2.58 (s, 3H), 2.54 (s, 3H), 2.38 (d, *J* = 13.7 Hz, 1H), 2.12 (d, *J* = 12.1 Hz, 2H), 2.05 - 1.83 (m, 6H), 1.72 - 1.61 (m, 2H).
MS [M+H]+ for C₃₀H₃₆³⁵ClN₄O₂ = 519.25
IR (KBr, cm-1): 3196, 2949,1686, 1448

### Synthesis of compound Comparative example:

### Synthesis of 6-chloro-1,4-dimethyl-9-(oxiran-2-ylmethyl)-3-pyrrol-1-yl-carbazole :

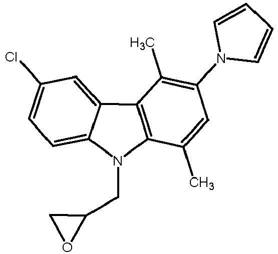

A solution of 6-chloro-1,4-dimethyl-3-pyrrol-1-yl-9*H*-carbazole (I-a) (0.50 g, 1.69 mmol) and crushed potassium hydroxide pellets (0.11 g, 2.03 mmol) in DMF (3 ml) was stirred for an hour before the addition of epichlorohydrin (0.33 ml, 4.24 mmol). The reaction was stirred overnight at room temperature. The mixture was worked up by dilution with EtOAc (20 ml), and washed two times with water (10 ml) and then brine. The organic layer was dried over MgSO₄, filtered and evaporated in vacuo. The crude product is purified on silica gel with gradient of elution cyclohexane to cyclohexane / DCM 8/2. The compound expected was obtained as a yellow solid in 73% yield.
¹H NMR (400 MHz, CDCl₃) δ 8.09 (d, *J* = 1.9 Hz, 1H), 7.39 (dd, *J* = 8.8, 1.9 Hz, 1H), 7.35 (d, *J* = 8.8 Hz, 1H), 7.11 (s, 1H), 6.75 (t, *J* = 2.1 Hz, 2H), 6.27 (t, *J* = 2.1 Hz, 2H), 4.95 (dd, *J* = 16.4, 2.3 Hz, 1H), 4.59 (dd, *J* = 16.5, 4.2 Hz, 1H), 3.32 (tt, *J* = 4.2, 2.4 Hz, 1H), 2.77 - 2.70 (m, 4H), 2.50 (s, 3H), 2.35 (dd, *J* = 4.7, 2.6 Hz, 1H).
¹³C NMR (101 MHz, CDCl₃) δ 140.5, 138.8, 133.0, 128.8, 127.5, 125.7, 125.3, 124.8, 123.1 (2C), 122.3, 121.9, 117.8, 110.2, 108.5 (2C), 51.3, 45.3, 44.7, 20.2, 15.1.

### Synthesis of N-[3-(6-chloro-1,4-dimethyl-3-pyrrol-1-yl-carbazol-9-yl)-2-hydroxypropyl]-1,1,1-trifluoro-N-(3-methoxyphenyl)methanesulfonamide:

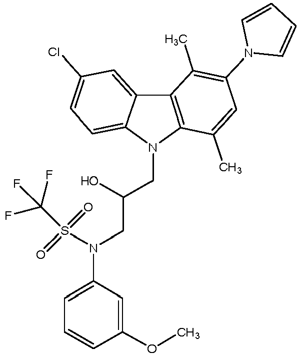

N-BuLi (2.5 M in hexanes, 0.21 ml) was added dropwise to an ice-cooled solution of 1,1,1-trifluoro-N-(3-methoxyphenyl)methanesulfonamide (0.09g, 0.37 mmol) in dry dioxane (3 ml). The solution was then stirred at room temperature for 15 minutes before addition of 6-chloro-1,4-dimethyl-9-(oxiran-2-ylmethyl)-3-pyrrol-1-yl-carbazole (0.10 g, 28.5 mmol), followed by heating at 90°C for two hours. The reaction was allowed to cool to room temperature then diluted with EtOAc (20 ml) and washed two times with water and finally brine. The organic layer was dried over MgSO₄, filtered and evaporated in vacuo. The crude product is purified on silica gel with gradient of elution cyclohexane to cyclohexane / EtOAc 8/2. The desired compound was obtained as a yellow oil in 72% yield.
¹H NMR (400 MHz, CDCl₃) δ 8.07 (d, *J* = 2.0 Hz, 1H), 7.33 (dd, *J* = 8.7, 2.0 Hz, 1H), 7.22 (dd, *J* = 8.4, 3.6 Hz, 2H), 7.07 (s, 1H), 6.86 (dd, *J* = 8.6, 2.4 Hz, 2H), 6.79 (t, *J* = 2.3 Hz, 1H), 6.73 (t, *J* = 2.1 Hz, 2H), 6.27 (t, *J* = 2.1 Hz, 2H), 4.60 - 4.46 (m, 2H), 4.18 - 4.14 (m, 1H), 4.01 - 3.85 (m, 2H), 3.72 (s, 3H), 2.63 (s, 3H), 2.48 (s, 3H).
¹³C NMR (101 MHz, CDCl₃) δ 160.4, 139.9, 138.4, 137.8, 133.4, 130.5, 129.0, 127.6, 125.8, 125.6, 125.2, 123.1 (2C), 122.5, 122.2, 121.9, 120.4, 117.5, 115.2, 114.9, 114.1 (q, *J*= 245.1 Hz),109.9, 108.6 (2C), 55.6, 54.7 (d, *J*= 22.4 Hz), 46.4 (d, *J*= 22.7 Hz), 20.4, 15.1.

### Synthesis of N-[3-(6-chloro-1,4-dimethyl-3-pyrrol-1-yl-carbazol-9-yl)-2-fluoro-propyl]-1,1,1-trifluoro-N-(3-methoxyphenyl)methanesulfonamide

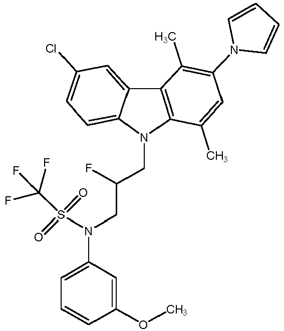

Morpho-Dast (0.35 ml, 0.03 mmol) was added to a solution of N-[3-(6-chloro-1,4-dimethyl-3-pyrrol-1-yl-carbazol-9-yl)-2-hydroxy-propyl]-1,1,1-trifluoro-N-(3-methoxyphenyl)methanesulfonamide (0.50 g, 0.82 mmol) in anhydrous DCM (8 ml) and stirred overnight at room temperature. The solution, in a water bath at ambient temperature, was neutralized by dropwise addition of 3.75 ml saturated bicarbonate solution. The biphasic mixture was extracted with DCM twice. The combined organics were dried over MgSO₄, filtered and condensed. The crude product is purified on silica gel with gradient of elution cyclohexane to cyclohexane / EtOAc 8/2. The desired compound was obtained as a yellow solid in 74% yield.
¹H NMR (400 MHz, CDCl₃) δ 8.18 (d, *J* = 2.0 Hz, 1H), 7.45 (dd, *J* = 8.8, 2.1 Hz, 1H), 7.37 - 7.31 (m, 1H), 7.29 (m, 1H), 7.17 (s, 1H), 6.99 - 6.92 (m, 2H), 6.89 (t, *J* = 2.3 Hz, 1H), 6.83 (t, *J* = 2.1 Hz, 2H), 6.36 (t, *J* = 2.1 Hz, 2H), 4.94 - 4.79 (m, 2H), 4.79 - 4.67 (m, 1H), 4.26 - 4.03 (m, 2H), 3.81 (s, 3H), 2.70 (s, 3H), 2.58 (s, 3H).
¹³C NMR (101 MHz, CDCl₃) δ 160.6, 139.9, 138.5, 138.0, 133.2, 130.4, 128.2, 127.4, 125.6, 125.3, 125.0,123.0 (2C), 122.4, 122.0, 120.2, 117.9, 114.8, 114,7, 114.1 (q, *J*= 245.1 Hz) 110.3, 108.5 (2C), 90.5 (d, *J*= 182 Hz), 69.8, 56.3 (d, *J*= 21.7 Hz), 48.2 (d, *J*= 21.7 Hz), 20.4, 15.1.

### Synthesis of N-[3-(6-chloro-1,4-dimethyl-3-pyrrol-1-yl-carbazol-9-yl)-2-fluoro-propyl]-3-methoxy-aniline

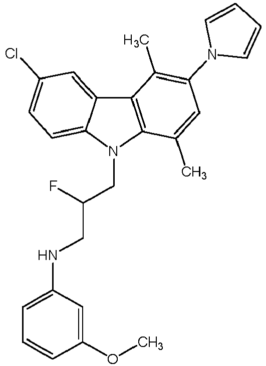

A two neck flask containing N-[3-(6-chloro-1,4-dimethyl-3-pyrrol-1-yl-carbazol-9-yl)-2-fluoro-propyl]-1,1,1-trifluoro-N-(3-methoxyphenyl)methanesulfonamide (0.20 g, 0.328 mmol) was purged with N₂ before addition of degassed xylene (3 ml). The solution was cooled in a dry-ice acetone bath before the dropwise addition of Red-Al^{®} (sodium bis(2-methoxyethoxy)aluminium hydride solution, 65% wt in toluene, 0.44 ml, 1.53 mmol) during which the internal temperature was maintained between -50 to -40°C. The cold bath was removed immediately upon completion of Red-AI addition. The reaction was allowed to warm slowly to about -20°C at which time, the reaction flask was heated until the internal temperature was 60°C. Heating was continued for an hour, and then the mixture was allowed to cool to ambient temperature over 30 minutes. The reaction mixture was diluted with EtOAc and washed with water twice. The organic layer was dried over MgSO₄, filtered and condensed. The crude product is purified on silica gel with gradient of elution cyclohexane to cyclohexane / EtOAc 8/2. The desired compound was obtained as a yellow solid in 50% yield.
¹H NMR (400 MHz, CDCl₃) δ 8.10 (d, *J* = 2.0 Hz, 1H), 7.34 (dd, *J* = 8.7, 2.0 Hz, 1H), 7.29 (d, *J* = 8.9 Hz, 1H), 7.09 (s, 1H), 7.02 (t, *J* = 8.1 Hz, 1H), 6.74 (t, *J* = 2.3 Hz, 2H), 6.27 (p, *J* = 3.0 Hz, 2H), 6.14 (dd, *J* = 8.2, 2.2 Hz, 1H), 6.06 (t, *J* = 2.4 Hz, 1H), 4.98 (m, 1H), 4.87 (dd, *J* = 15.9, 7.5 Hz, 1H), 4.73 (dd, *J* = 15.9, 4.1 Hz, 1H), 3.90 (t, *J* = 6.4 Hz, 1H), 3.42 - 3.24 (m, 2H), 2.68 (s, 3H), 2.50 (s, 3H).
¹³C NMR (101 MHz, CDCl₃) δ 160.9, 148.5, 140.1, 138.5, 133.2, 130.3, 129.0, 127.6, 125.7, 125.4, 125.1, 123.1(2C), 122.5, 122.2, 117.8, 110.2, 108.5 (2C), 106.3, 103.7, 99.5, 91.5 (d, *J*= 180 Hz), 55.1, 46.4 (d, *J*=22.3Hz), 45.7 (d, *J*=22.3Hz), 20.3, 15.1.

### Example 2: Quantitative analysis of the neuroprotective effect of compound I-a and I-s

### Isolation of adult DRG neurons

Dorsal root ganglia (DRG) were dissected from 8- to 10-wk-old C57BL/6J mice in cold Hank's balanced salt solution (HBSS) (Life Technologies) or Dulbecco's Modified Eagle's medium (Life Technologies) and dissociated in 1 mg/mL Collagenase A for 1 h at 37°C, followed by 0.05% trypsin (Life Technologies) digestion for 3 to 5 min at 37°C and washed with Neurobasal medium (Invitrogen) supplemented with 2% B-27 (Invitrogen), 0.5 mM glutamine (Invitrogen), fetal bovine serum (FBS), and 100 U/mL penicillin-streptomycin. DRG neurons were then triturated by repeated gentle pipetting until no clump was visible, and neuronal bodies were resuspended in Neurobasal medium with FBS prior to plating onto 12 well plates (over 18 mm coverslips) that had been coated overnight with 100 µg/mL poly-D-lysine at 37°C and for 1 h at 37°C with 10 µg/mL laminin (Life Technologies). After 30 min, Neurobasal medium, without FBS, was added to the plate. At 4 days in vitro (DIV), at least 30% of media was changed and 10 µM Cytosine β-D-arabinofuranoside hydrochloride (Sigma) was added to media every 4 days.

### Treatments of dissociated DRG neurons

Dissociated adult DRG neurons prepared as described above were treated for 72h by 50 nM paclitaxel (PTX), 12 µM compound I-a, 1µM compound I-s or their combination, as indicated, fixed and immunostained with mouse antineurofilament antibodies before measurement of the degeneration ilndex.

### Degeneration Index Measurements in DRG Neurons.

Images of random fields of dissociated adult DRG neurons fixed and immunostained with mouse antineurofilament antibodies were acquired using a 20Å~ objective lens (Olympus IX81) coupled to a monochrome charge-coupled device camera (Sensicam QE; Cooke Corporation). To quantify axonal degeneration, the areas occupied by the axons (total axonal area) and degenerating axons (fragmented axonal area) were measured in the same field from images of DRG neurons. Images were automatically thresholded (global threshold) using a default autothreshold method, binarized, and the fragmented axonal area measured by using the particle analyzer module of imaged (size of small fragments = 20 to 10,000 pixels). Degeneration index was calculated as the ratio between the fragmented axonal area and the total axonal area.

Images of random fields of dissociated adult DRG neurons, treated for 72h by PTX (50 nM), compound I-a (12 µM), compound I-s (1 µM) or their combination, as indicated, fixed and immunostained with mouse anti-neurofilament antibodies, were acquired using a 20Å~ objective lens (Olympus IX81) coupled to a monochrome charge-coupled device camera (Sensicam QE; Cooke Corporation).

To quantify axonal degeneration, the areas occupied by the axons (total axonal area) and degenerating axons (fragmented axonal area) were measured in the same field from images of DRG neurons. Degeneration index was calculated as the ratio between the fragmented axonal area and the total axonal area.

### Example 3: Mechanism behind the neuroprotective effect of the compounds of formula I

The mechanism behind the neuroprotective effect of this series of compounds has been identified. It has been demonstrated their neuroprotective effect results from activation of a metabolic enzyme: nicotinamide phosphoribosyl transferase (NAMPT), most probably. NAMPT is a key enzyme in the nicotinamide adenine dinucleotide (NAD) salvage pathway, playing a central role in maintaining the cellular NAD pool. Recent studies indicate that NAD is a central modulator controlling the health of damaged or diseased neurons.

The inventors have shown that I-a activates NAMPT in vitro, in a biochemical assay, but also in cells, in two different assays. The first measures the kinetics of endogenous NADH production by confocal microscopy after UV excitation (351-364 nm) and measurement of NADH autofluorescence at 390-486 nm. A dose-dependent increase in NADH production after I-a and I-s treatment was measured (see figure 2).

In another assay, it was demonstrated that the series of compounds can counteract NAMPT inhibition by FK866. The compound FK866 is known to be a potent selective inhibitor of the NAMPT enzyme. Administration of FK866 in cultured cells results in depletion of intracellular NAD levels and subsequent cell death. The effect of compound I-a and other compounds of the series on FK866-induced cytotoxicity in HeLa cells was assayed.

HeLa cells were seeded in microplates and incubated with 3.5 nM FK866 without (black) or in the presence of 6 µM of I-a and different analogues (grey), as indicated, for 72 hours. A viability assay (prestoblue assay) was then conducted.

While FK866 induced cell death of over 90% of cells, leaving only 8% alive cells (Y-axis), co-incubation of this inhibitor with compounds I-a, I-f, I-m, I-n and I-q reduced cell death. Compound I-m had no detectable protective effect. These results show that compounds I-a, I-f, I-m, I-n and I-q can relieve the cytotoxicity of the NAM PT inhibitor FK866, strongly suggesting that activation of NAMPT is involved in the protective activity of these compounds. Of note, the comparative example does not reduce FK866-induced cell death.

### Example 4: prevention of paclitaxel-induced neuropathy by compound I-a in the rat

The neuroprotection evidenced in vitro in the example 2 also occurs in-vivo, in a rat model of neuropathy.

In this model, injections of 4 mg/mL of PTX (white triangle) progressively induce a tactile hypersensitivity, assessed by results from the electronic von Frey assay, which measures the maximum force applied (expressed in grams, Y-axis) to induce paw withdrawal. Such a hypersensitivity does not occur on rats treated with the vehicle, with compound I-a (50mg/kg), alone (black dots) or in combination with PTX (black triangles). This example demonstrates that compound I-a has no hypersensitive effect in itself and prevents the hypersensitivity induced by PTX.

## Claims

1. A compound of formula (I) 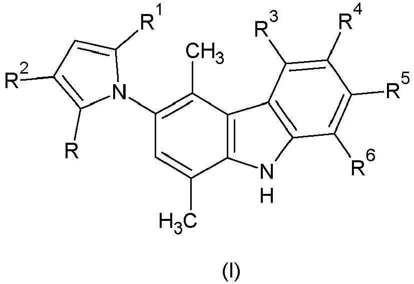
wherein R, R¹ and R² are independently selected form the group consisting of:
- a hydrogen atom,
- a halogen atom,
- an alkyl group, linear, cyclic or branched, saturated or unsaturated, possibly substituted, comprising from 1 to 10 carbon atoms,
- an acyl group comprising from 1 to 10 carbon atoms,
- a carboxyl group,
- an amido group comprising from 1 to 10 carbon atoms, and
- an imino group, possibly substituted by an alkyl group, linear, cyclic or branched, saturated or unsaturated, and
wherein R³, R⁴, R⁵ and R⁶ are independently selected form the group consisting of:
- a hydrogen atom,
- a halogen atom,
- a hydroxyl group,
- an alkyl group, linear, cyclic or branched, saturated or unsaturated, possibly substituted, comprising from 1 to 10 carbon atoms,
- an alkoxy group comprising from 1 to 10 carbon atoms,
- an acyl group comprising from 1 to 10 carbon atoms,
- a carbonate group from 1 to 10 carbon atoms,
- a carboxyl group, and
- a cyano group
for use as neuroprotectants, preferably by preventing peripheral neurons from degenerating after chemotherapy, or in the treatment of defects observed in disorders with a reduced NAD metabolism, preferably chosen from the following:
- central and peripheral neurodegenerative diseases such as Alzheimer's disease, Parkinson's disease, Huntington's disease, Amyotrophic Lateral Sclerosis disease, Guillain-Barré syndrome;
- neuronal trauma, including noise-induced hearing loss;
- metabolic diseases such as diabetes or obesity;
- organ damage/failure such as liver damage/failure, kidney damage/failure or heart damage/failure;
- certain muscular diseases such as Duchene disease;
- ageing;
- metabolic defects affecting sperm mobility;
- the oxidative damage during virus infection.

2. The compound of formula (I) for use according to claim 1, wherein R, R¹ and R² are independently selected from the group consisting of:
- a hydrogen atom,
- a halogen atom,
- an alkyl group of formula -CₙH₂ₙ₊₁₋ₓXₓ, X being selected from the group consisting of a hydrogen atom, a halogen atom, a hydroxyl group, and n being an integer between 1 and 10 and x being the number of X present in the alkyl group, and preferably x being 1 and X being in terminal position in the alkyl group,
- an alkyl group bearing an amino (primary, secondary, or tertiary),
- an alkyl group bearing an ester, the ester being preferably an ester of a protected (e.g. Boc, Fmoc or Cbz protected) amino acid such as lysine, valine, tyrosine, cysteine, glutamic acid, an ester of a diacid, such as succinic acid or an ester of acid bearing a polyethylene glycol, having from 4 to 50 repeating units,
- an alkyl group bearing piperidine or a dipiperidine linked via a carbamate,
- an acyl group of formula -C(O)ORₐ, Rₐ being a linear and saturated alkyl group comprising from 1 to 9 carbon atoms,
- an acyl group of formula -C(O)H,
- a carboxyl group (-COOH),
- an amido group of formula -C(O)NR_{b}, R_{b} being a linear and saturated alkyl group comprising from 1 to 9 carbon atoms, and
- an imino group of formula -C=N-R_{c}, R_{c} being selected from the group consisting of a hydrogen atom or an alkyl group, linear, saturated and comprising from 1 to 10 carbon atoms, or a phenyl group, and
wherein R³, R⁴, R⁵ and R⁶ are independently selected from the group consisting of:
- a hydrogen atom,
- a halogen atom,
- a hydroxyl group,
- an alkyl group of formula -CₙH₂ₙ₊₁, linear, saturated and n being an integer between 1 and 10,
- an alkoxy group of formula -OR_{d}, R_{d} being a linear and saturated alkyl group comprising from 1 to 9 carbon atoms,
- an acyl group of formula -C(O)ORₐ, Rₐ being a linear and saturated alkyl group comprising from 1 to 9 carbon atoms,
- an acyl group of formula -C(O)Rₑ, Rₑ being a hydrogen atom or an alkyl group, linear, cyclic or branched, saturated or unsaturated, possibly substituted, comprising from 1 to 10 carbon atoms,
- a carbonate group from 1 to 10 carbon atoms,
- a carboxyl group (-COOH), and
- a cyano group (-CN).

3. The compound of formula (I) for use according to claim 1 or 2, wherein R and R¹ are independently selected from the group consisting of:
- a hydrogen atom,
- a halogen atom,
- an alkyl group, linear, saturated and comprising from 1 to 10 carbon atoms,
wherein R² is selected from the group consisting of:
- a hydrogen atom,
- a halogen atom,
- an alkyl group of formula -CₙH₂ₙ₊₁₋ₓXₓ, X being selected from the group consisting of a hydrogen atom, a halogen atom, a hydroxyl group, and n being an integer between 1 and 10 and x being the number of X present in the alkyl group, and preferably x being 1 and X being in terminal position in the alkyl group,
- an alkyl group bearing an amino (primary, secondary, or tertiary),
- an alkyl group bearing an ester, the ester being preferably an ester of a protected (e.g. Boc, Fmoc or Cbz protected) amino acid such as lysine, valine, tyrosine, cysteine, glutamic acid, an ester of a diacid, such as succinic acid or an ester of acid bearing a polyethylene glycol, having from 4 to 50 repeating units,
- an alkyl group bearing piperidine or a dipiperidine linked via a carbamate,-an acyl group of formula -C(O)ORₐ, Rₐ being a linear and saturated alkyl group comprising from 1 to 9 carbon atoms,
- an acyl group of formula -C(O)H,
- a carboxyl group (-COOH),
- an amido group of formula -C(O)NR_{b}, R_{b} being a linear and saturated alkyl group comprising from 1 to 9 carbon atoms, and
- an imino group of formula -C=N-R_{c}, R_{c} being selected from the group consisting of a hydrogen atom or an alkyl group, linear, saturated and comprising from 1 to 10 carbon atoms, or a phenyl group,
wherein R³, R⁵ and R⁶ are independently selected from the group consisting of:
- a hydrogen atom,
- a halogen atom,
- an alkyl group of formula -CₙH₂ₙ₊₁, linear, saturated and n being an integer between 1 and 10, and
wherein R⁴ is selected from the group consisting of:
- a hydrogen atom,
- a halogen atom,
- a hydroxyl group,
- an alkyl group of formula -CₙH₂ₙ₊₁, linear, saturated and n being an integer between 1 and 10,
- an alkoxy group of formula -OR_{d}, R_{d} being a linear and saturated alkyl group comprising from 1 to 9 carbon atoms,
- an acyl group of formula -C(O)ORₐ, Rₐ being a linear and saturated alkyl group comprising from 1 to 9 carbon atoms,
- an acyl group of formula -C(O)Rₑ, Rₑ being a hydrogen atom or an alkyl group, linear, cyclic or branched, saturated or unsaturated, possibly substituted, comprising from 1 to 10 carbon atoms,
- a carbonate group of formula -OC(O)OR_{f}, R_{f} being a linear and saturated alkyl group comprising from 1 to 9 carbon atoms,
- a carboxyl group (-COOH), and
- a cyano group (-CN).

4. The compound of formula (I) for use according to any one of claims 1 to 3,
wherein R and R¹ are independently selected from the group consisting of a hydrogen atom, a chloride atom and a methyl group,
wherein R² is selected from the group consisting of a hydrogen atom, a methyl group, a -COH group, an alkyl group of formula -CH₂X, X being selected from the group consisting of a chloride atom, a bromide atom, a iodine atom and a hydroxyl group, an acyl group of formula -C(O)ORₐ, Rₐ being a methyl group or an ethyl group, an acyl group of formula -C(O)H, a carboxyl group (-COOH), an amido group of formula -C(O)NR_{b}, R_{b} being a methyl group or an ethyl group, and an imino group of formula -C=N-R_{c}, R_{c} being a phenyl group, -CH₂Y, Y being selected from the group consisting of an amino group of formula -NR_{z}R_{z}, R_{z} independtly representing H or a C1 to C3 alkyl group, an ester, the ester being preferably an ester of a protected (e.g. Boc protected) amino acid such as lysine, valine, tyrosine, cysteine, glutamic acid, an ester of a diacid, such as succinic acid or an ester of acid bearing a polyethylene glycol, having from 4 to 50 repeating units, or a piperidine or a dipiperidine linked via a carbamate,
wherein R³, R⁵ and R⁶ are independently selected from the group consisting of a hydrogen atom, a chloride atom, a methyl group and an ethyl group, and
wherein R⁴ is selected from the group consisting of a hydrogen atom, a methyl group, a hydroxyl group, a chloride atom, a bromide atom, a fluoride atom, a iodine atom, an alkoxy group of formula -OR_{d}, R_{d} being a methyl group or an ethyl group, an acyl group of formula -C(O)ORₐ, Rₐ being a methyl group or an ethyl group, an acyl group of formula -C(O)Rₑ, Rₑ being a methyl group, an ethyl group, a linear propyl group, a linear pentyl group, -CH₂CH₂-cyclopentyl group and a (*para*methyl)phenyl group, a carbonate group of formula -OC(O)OR_{f}, R_{f} being a methyl group or an ethyl group, a carboxyl group (-COOH), and a cyano group (-CN).

5. The compound of formula (I) for use according to any one of claims 1 to 4,
wherein R and R¹ are independently selected from the group consisting of a hydrogen atom and a methyl group,
wherein R² is selected from the group consisting of a hydrogen atom, a -COH group, a -CH₂OH group, a -CH₂NHC₂H₅ group, a -CH₂N(C₂H₅)₂ group, a -CH₂O-ValBoc group, a -CH₂O-(Boc)LysBoc group, a -CH₂OOC(CH₂)₂COOH group, a - CH₂OCO(PEG)-OCH₃ group and a -CH₂OCO(NC₅H₉)NC₅H₁₀ group,
wherein R³, R⁵ and R⁶ are independently selected from the group consisting of a hydrogen atom, a chloride atom and an ethyl group, and
wherein R⁴ is selected from the group consisting of a hydrogen atom, a hydroxyl group, a chloride atom, a bromide atom, a fluoride atom, a carbonate group of formula -OC(O)OR_{f}, R_{f} being an ethyl group and an acyl group of formula - C(O)ORₐ, Rₐ being a methyl group.

6. The compound of formula (I) for use according to any one of claims 1 to 5, **characterized in that** at least three of the substituents selected from the group consisting of R³, R⁴, R⁵ and R⁶ are a hydrogen atom.

7. The compound of formula (I) for use according to any one of claims 1 to 6, **characterized in that** said compound is selected from the group consisting of

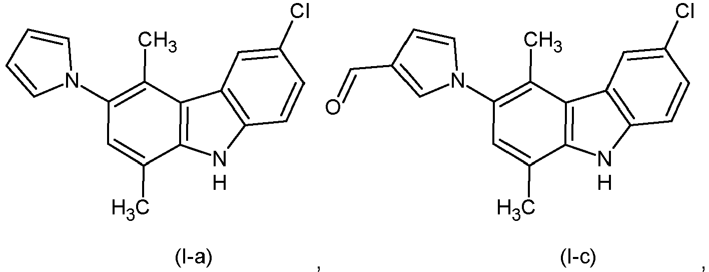

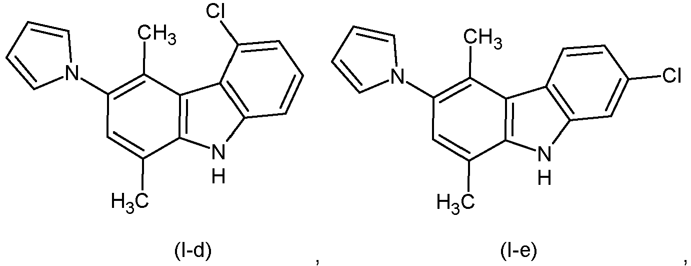

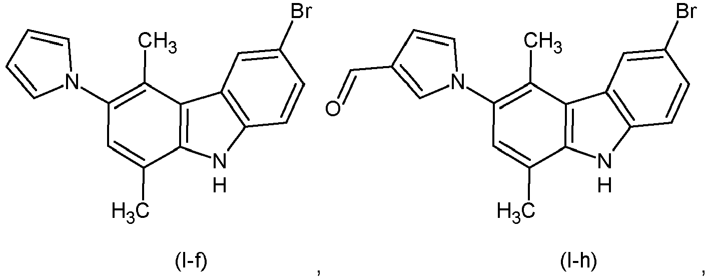

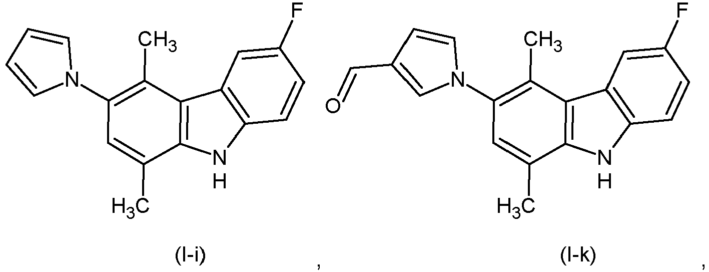

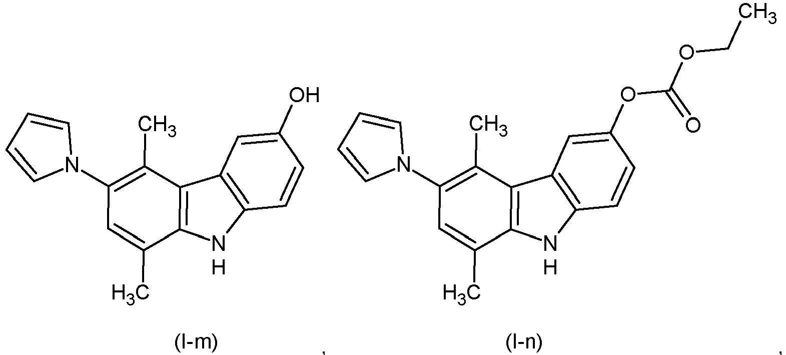

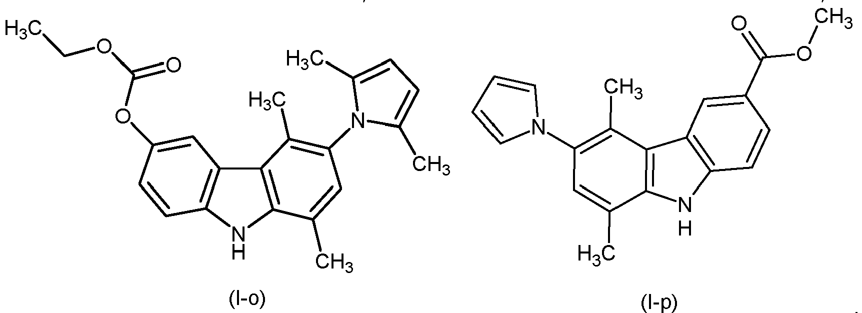

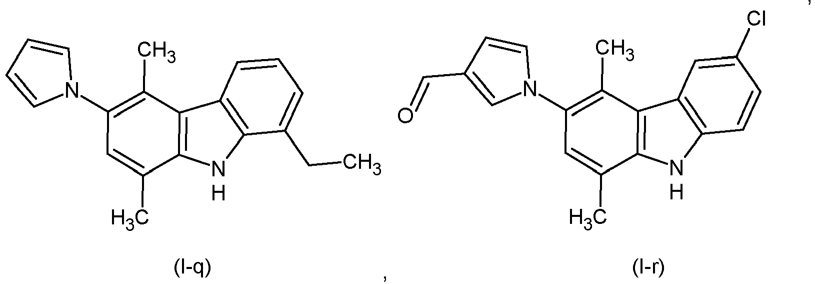

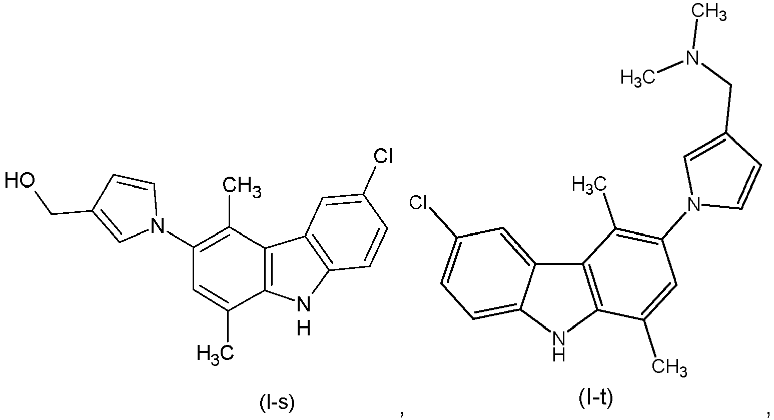

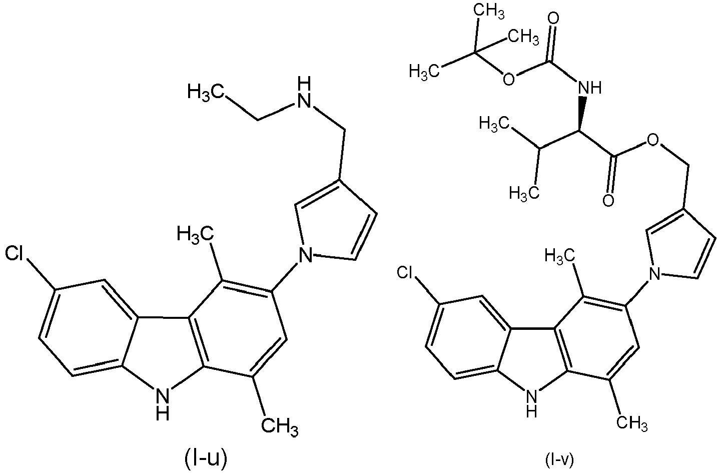

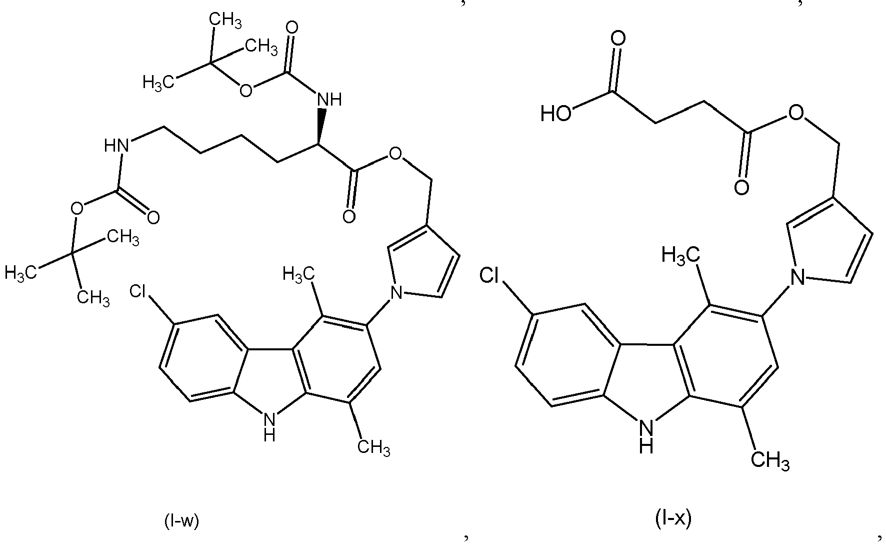

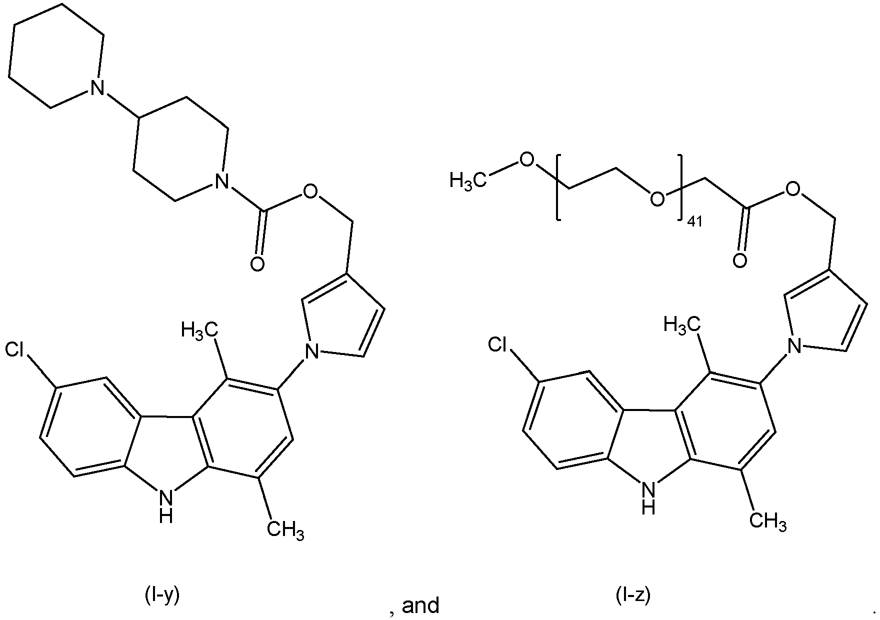

8. A pharmaceutical composition comprising at least one compound of formula (I) according to any one of claims 1 to 7 and at least one pharmaceutically acceptable excipient, for use as neuroprotectants.

9. A pharmaceutical composition comprising at least one compound of formula (I) according to any one of claims 1 to 7 and at least one pharmaceutically acceptable excipient, for use in the treatment of defects observed in disorders with a reduced NAD metabolism.

10. The pharmaceutical composition for use according to any of claims 8 to 9, said composition being in the form of hard gelatin capsules, of capsules, of compressed tablets, of suspensions, of lozenges or of injectable solutions, ointments.

11. The compound of formula (I) for use according to any of claims 1 to 7 or the pharmaceutical composition for use according to any of claims 8 to 10, said compound or composition being administered to a patient, the patient being human or animal, preferably by oral, buccal, inhalation, sublingual, nasal, percutaneous, i.e. transdermal or parenteral, including intravenous, intramuscular, subcutaneous and intracoronary, administration.

12. The compound of formula (I) for use according to any of claims 1 to 7 or the pharmaceutical composition for use according to claims 8 to 11, wherein the compound is in the form of its tautomeric, racemic, enantiomeric or polymorphic forms or pharmaceutically- acceptable salts.

13. The compound of formula (I) for use according to any of claims 1 to 7 or the pharmaceutical composition for use according to claims 8 to 12, wherein the compound is in the form of a co-crystal, said co-crystal comprising the compound of formula (I) and a co-former, the co-former being preferably chosen from nicotinamide, isonicotinamide, nicotinamide riboside, nicotinamide mononucleotide, ascorbic acid, syringic acid, gallic acid and caffeine.

14. A co-crystal comprising one compound of formula (I) as defined in any one of claims 1 to 7 and a co-former, the co-former being preferably chosen from nicotinamide, isonicotinamide, nicotinamide riboside, nicotinamide mononucleotide, ascorbic acid, syringic acid, gallic acid, caffeine.
